# EUROPEAN PATENT SPECIFICATION

(11) **EP 3 268 037 B1**
(45) Date of publication and mention of the grant of the patent: **31.08.2022**
(21) Application number: 16711469.3
(22) Date of filing: 09.03.2016
(51) Int. Cl.: A61K 39/395, C07K 16/28

(54) **CD27 AGONISTS**
CD27 AGONISTS
AGONISTES CD27

(30) Priority: 09.03.2015 US 201562130325 P
(43) Date of publication of application: 17.01.2018
(73) Proprietor: Celldex Therapeutics, Inc., Needham, MA 02494-2725 (US)
(72) Inventor: KELER, Tibor, Pipersville, PA 18947-1613 (US); GOLDSTEIN, Joel, Hopewell, NJ 08525 (US); HE, Lizhen, Allentown, PA 18104 (US); MARSH, Henry, C., Reading, MA 01867 (US); SUNDARAPANDIYAN, Karuna, Kendall Park, NJ 08824 (US); THOMAS, Lawrence, J., Easton, MA 02375 (US); VITALE, Laura, A., Doylestown, PA 18902 (US); WIDGER, Jenifer, Alpha, NJ 08865 (US)
(74) Representative: J A Kemp LLP
(86) International application number: PCT/US2016/021569
(87) International publication number: WO 2016/145085

(56) References cited:
- WO-A1-2012/004367
- WO-A1-2015/016718
- Anonymous: "Abstract 1246: Development of an agonistic antibody against the human T-cell costimulatory receptor CD27 as a potential immunotherapeutic tool. | Cancer Research", , 15 April 2013 (2013-04-15), XP055283844, Retrieved from the Internet: URL:http://cancerres.aacrjournals.org/cont ent/73/8_Supplement/1246 [retrieved on 2016-06-27]
- ROSS STEWART ET AL: "The role of Fc gamma receptors in the activity of immunomodulatory antibodies for cancer", JOURNAL FOR IMMUNOTHERAPY OF CANCER, BIOMED CENTRAL LTD, LONDON, UK, vol. 2, no. 1, 19 August 2014 (2014-08-19) , page 29, XP021193931, ISSN: 2051-1426, DOI: 10.1186/S40425-014-0029-X
- CHERN SIANG LEE ET AL: "Novel antibodies targeting immune regulatory checkpoints for cancer therapy", BRITISH JOURNAL OF CLINICAL PHARMACOLOGY, vol. 76, no. 2, 23 July 2013 (2013-07-23), pages 233-247, XP055151740, ISSN: 0306-5251, DOI: 10.1111/bcp.12164
- KONTERMANN ROLAND E: "Dual targeting strategies with bispecific antibodies", MABS, LANDES BIOSCIENCE, US, vol. 4, no. 2, 1 March 2012 (2012-03-01), pages 182-197, XP002698995, ISSN: 1942-0862, DOI: 10.4161/MABS.4.2.19000

## Description

Interactions between T cells and antigen-presenting cells involve a variety of accessory molecules that facilitate in the generation of an immune response. One such molecule is CD27, which binds CD70 and belongs to the tumor necrosis factor receptor (TNF-R) superfamily (Ranheim, E.A. et al. (1995) Blood, 85(12):3556-65). CD27 typically exists as a glycosylated, type I transmembrane protein, frequently in the form of homodimers with a disulfide bridge linking the two monomers. The disulfide bridge is in the extracellular domain close to the membrane (Camerini et al. (1991) J. Immunol., 147:3165-69). CD27 may also be expressed in a soluble form (see, *e.g.,* van Oers, M.H. et al. (1993) Blood 82(11):3430-6 and Loenen, W.A. et al. (1992) Eur. J. Immunol., 22:447). Cross-linking the CD27 antigen on T cells provides a costimulatory signal that, in concert with T-cell receptor crosslinking, can induce T-cell proliferation and cellular immune activation.

CD27 is expressed on mature thymocytes, on most CD4+ and CD8+ peripheral blood T cells, natural killer cells and B cells (Kobata, T. et al. (1995) Proc. Natl. Acad. Sci. USA, 92(24): 11249-53). CD27 is also highly expressed on B cell non-Hodgkin's lymphomas and B cell chronic lymphocytic leukemias (Ranheim, E.A. et al. (1995) Blood, 85(12):3556-65). Additionally, increased levels of soluble CD27 protein have been identified in sera or sites of disease activity in parasitic infection, cytomegalovirus (CMV) infection, sarcoidosis, multiple sclerosis, and B-cell chronic lymphocytic leukemia (Loenen, W.A. et al. (1992) Eur. J. Immunol, 22:447).

Agonistic monoclonal antibodies against CD27 have recently been shown to promote T cell responses and show promise as anti-cancer therapeutics (see *e.g.,* Sakanishi, T. et al. (2010) Biochem Biophys. Res. Commun., 393:829-835 and PCT Publication WO 2008/051424). Anti-CD27 antibodies that are agonists are cross-linked through interaction of their Fc domain with Fc receptors (FcR) through cis or trans interactions, that is, cross-linking CD27 and FcR on the same or different cells. This requirement for FcR interaction of anti-CD27 agonist antibodies implies that the T cell stimulating activity is at least partially dependent on the number FcR expressing cells, which could be a limiting factor in their usefulness.

Accordingly, additional therapeutic agents that work through CD27, in particular CD27 agonists that lack the requirement of interacting with FcR, are still needed in the art.

WO 2015/016718 describes combining CD27 agonists and immune checkpoint inhibition for immune stimulation.

### Summary of the Invention

The invention provides CD27 agonists that comprise two components linked to each other. The first component is an anti-CD27 antibody, or antigen-binding fragment thereof, which is linked to a second component that is an anti-PD-L1 antibody, or antigen-binding fragment thereof. Preferably, the anti-PD-L1 antibody, or antigen-binding fragment thereof does not bind to an Fc receptor and the CD27 agonist stimulates T cell activity without the need for Fc receptor interaction.

In one embodiment, the second component is an anti-PD-L1 scFv. In one embodiment, the first component is an anti-CD27 antibody and the second component is an anti-PD-Ll scFv linked to the C-terminus of the heavy chain of the anti-CD27 antibody. In one embodiment, (i) the anti-CD27 antibody comprises a VH CDR3 shown in SEQ ID NO: 19 and a VL CDR3 shown in SEQ ID NO: 22 and (ii) the anti-PD-L1 scFv comprises a VH CDR3 shown in SEQ ID NO: 60 and a VL CDR3 shown in SEQ ID NO: 63. In another embodiment, (i) the anti-CD27 antibody comprises VH CDR1, CDR2 and CDR3 shown in SEQ ID NOs: 17, 18 and 19, respectively, and VL CDR1, CDR2 and CDR3 shown in SEQ ID NOs: 20, 21 and 22, respectively, and (ii) the anti-PD-L1 scFv comprises VH CDR1, CDR2 and CDR3 shown in SEQ ID NOs: 58, 59 and 60, respectively, and VL CDR1, CDR2 and CDR3 shown in SEQ ID NOs: 61, 62 and 63, respectively. In another embodiment, (i) the anti-CD27 antibody comprises VH and VL chains comprising the amino acid sequences shown in SEQ ID NOs: 1 and 2, respectively; and (ii) the anti-PD-L1 scFv comprises VH and VL chains comprising the amino acid sequences shown in SEQ ID NOs: 32 and 33, respectively. In another embodiment, the CD27 agonist comprising an anti-CD27 antibody and an anti-PD-L1 scFv comprises a heavy chain comprising the amino acid sequence shown in SEQ ID NO: 66 and a light chain comprising the amino acid sequence shown in SEQ ID NO: 67. In another embodiment, the heavy chain is encoded by the nucleotide sequence shown in SEQ ID NO: 64 and the light chain is encoded by the nucleotide sequence shown in SEQ ID NO: 65.

A CD27 agonist comprising a first component that is an anti-CD27 antibody, or antigen-binding fragment thereof, linked to a second component that is an anti-PD-L1 antibody, or antigen-binding fragment thereof, exhibits synergistic effects (e.g., in enhancing immune responses *in vivo*) as compared to use of anti-CD27 antibodies and anti-PD-L1 antibodies in combination (i.e., co-administration of unlinked antibodies).

The CD27 agonists may or may not contain any functional Fc receptor-binding domains.

A CD27 agonist of the invention can be, for example, a fusion protein, which can be made by genetic engineering using standard recombinant DNA techniques to operatively link nucleic acid encoding the first and second components. Alternatively, a CD27 agonist of the invention can be a chemical conjugate, which can be made by chemical conjugation of the first and second components.

In another aspect, the invention pertains to pharmaceutical compositions comprising a CD27 agonist of the invention and a pharmaceutically acceptable carrier.

In yet another aspect, the invention pertains to an *in vitro* method of stimulating T cell activity comprising contacting T cells with a CD27 agonist of the invention. Stimulating T cell activity can comprise, for example, stimulating IFN-gamma production. In yet another aspect, the invention pertains to a CD27 agonist of the invention for use in a method for inducing or enhancing an immune response, e.g., against an antigen, in a subject. The subject can be, for example, one who suffers from a condition or disease in which stimulation of an immune response is desired. A preferred condition or disease in which stimulation of an immune response is desired is cancer. The method of inducing or enhancing an immune response, e.g., against an antigen, in a subject can further comprise administering the antigen to the subject. Preferred antigens to be co-administered with a CD27 agonist of the invention are tumor antigens.

### Brief Description of the Drawings

Figure 1 is a schematic diagram of exemplary CD27 agonists that are CD27 hyper-crosslinking agents constructed either genetically or by chemical conjugation.
Figure 2 is a schematic diagram of exemplary CD27 agonists that are multi-specific agents.
Figure 3 is a photograph of an SDS-PAGE gel showing anti-CD27 (1F5) multimer pools created by chemical conjugation of full-length 1F5 antibody with 1F5 antibody Fab' fragments.
Figures 4A-4C are schematic diagrams of exemplary expression vectors for CD27 agonists. Figure 4A shows the vector pDGV1F5-sFv. Figure 4B shows the vector pDGV1F5-ScFvs. Figure 4C shows the vector pDGV1F5-CD70ECD.
Figure 5 is photograph of an SDS-PAGE gel of genetically engineered CD27 agonists under reducing and non-reducing conditions. Lane 1 shows 1F5 mAb. Lane 2 shows 2C2 mAb. Lane 3 shows 1F5mAb-1F5sFv. Lane 4 shows 1F5mAb-2C2sFv. Lane 5 shows 1F5mAb-CD70.
Figure 6 is photograph of an SDS-PAGE gel of genetically engineered multi-specific CD27 agonists under reducing and non-reducing conditions. Lane 1 shows 1F5mAb-1F5sFv. Lane 2 shows 1F5mAb-αPDL1sFv.
Figures 7A-7B are graphs illustrating the binding of multi-specific CD27 agonists to human CD27 (Figure 7A) and to human and mouse PD-L1 (Figure 7B).
Figures 8A-8B are graphs illustrating the binding of CD27 agonists to soluble recombinant CD27 (sCD27; Figure 8A) and to sCD27 having an R87A substitution (Figure 8B).
Figure 9 is a bar graph showing enhanced T cell activation by CD27 agonists, as measured by increased IFN-γ production.
Figure 10 is a graph showing T cell activation by CD27 agonists, as measured by increased IFN-γ production.
Figure 11 is a graph showing T cell activation by CD27 agonists, as measured by increased IL-2 production.
Figure 12 is a graph showing stimulation of NFκB by CD27 agonists using a CD27 expressing reporter cell line
Figure 13 is a graph showing stimulation of NFκB by CD27 agonists using a CD27 expressing reporter cell line.
Figure 14 is a bar graph showing *in vivo* T cell activation by CD27 agonists in a human CD27 transgenic mouse model.
Figure 15 is a graph showing *in vivo* anti-tumor activity of the 1F5mAb-αPDL1sFv in human CD27 transgenic mice transplanted with BCL1 lymphoma cells.
Figures 16A-D are graphs showing immune correlates in mice inoculated with BCL1 cells and treated with saline, anti-PD-L1 antibody, anti-CD27 antibody, anti-PD-L1 and anti-CD27 antibodies in combination or 1F5 mAb x anti-PD-L1 scFv bispecific agent. Figure 16A shows tumor (spleen) weight. Figure 16B shows % BCL1 cells in the spleen. Figure 16C shows % CD3⁺ T cells among non-BCL1 splenocytes. Figure 16D shows % CD4⁺ or CD8⁺ non-BCL1 splenocytes.
Figure 17 is a graph showing percent survival over time of mice inoculated with BCL1 cells and treated with an isotype control antibody, anti-PD-L1 antibody, anti-CD27 antibody, anti-PD-L1 and anti-CD27 antibodies in combination or 1F5 mAb x anti-PD-L1 scFv bispecific agent.

### Detailed Description

CD27 is an important co-stimulatory receptor that can be exploited for immunotherapy using agonist molecules. CD27 plays a key role in diverse immunological processes, including the survival, activation and effector functions of T cells, as well as the proliferation and cytotoxic activity of natural killer (NK) cells. These events occur in response to appropriate interaction of the ligand (CD70) with CD27 resulting in intracellular signaling events that lead to an activation of NF-kB and expression of relevant genes. As with most co-stimulatory molecules, effective stimulation of T cells with either the ligand or agonist antibodies also requires simultaneous stimulation through the T cell receptor (TCR).

Most CD27 agonists molecules require multimerization or crosslinking for their activity. For example, anti-CD27 antibodies that are agonists are cross-linked through interaction of their Fc domains with Fc receptors through cis or trans interactions. *In vitro* this can also be performed artificially using a secondary anti-Ig antibody or by adsorbing the antibody to a solid phase matrix. The requirement for FcR interaction of anti-CD27 agonist antibodies implies that the T cell stimulating activity is at least partially dependent on the number FcR expressing cells.

To eliminate the requirement for FcR interactions, CD27 agonists have been invented that 1) eliminate the need for interaction with receptors other than CD27 (herein termed CD27 hyper-crosslinking agents), or 2) can provide the crosslinking through interaction of alternate receptors that may be on different specific cell types not expressing FcR, and which alternate receptors may also provide additional function (herein termed CD27 multi-specific agents).

The invention provides CD27 agonists that comprise an anti-CD27 antibody, or antigen-binding fragment thereof, linked to an anti-PD-Ll antibody, or antigen-binding fragment thereof. Such anti-CD27 x anti-PD-Ll bispecific agents of the invention have now been shown to exhibit synergistic effects *in vivo,* such as in enhancing immune parameters and inhibiting tumor growth, as compared to co-administration of an anti-CD27 antibody with an anti-PD-L1 antibody (see Examples 9 and 10).

In order that the present invention may be more readily understood, certain terms are first defined. Additional definitions are set forth throughout the detailed description.

### I. Definitions

The term "CD27" (also referred to as "CD27 molecule", "CD27L receptor", "S1521", "T cell activation antigen CD27", "TNFRSF7," "MGC20393," "tumor necrosis factor receptor superfamily, member 7", "T cell activation antigen S152" "Tp55", "Tumor necrosis factor receptor superfamily member 7", "CD27 antigen", and "T-cell activation antigen CD27") refers to a receptor that is a member of the TNF-receptor superfamily, which binds to ligand CD70. CD27 is required for generation and long-term maintenance of T cell immunity and plays a key role in regulating B-cell activation and immunoglobulin synthesis. The term "CD27" includes any variants or isoforms of CD27 which are naturally expressed by cells (*e.g*., human CD27 deposited with GENBANK^{®} having accession no. AAH12160.1). Accordingly, CD27 agonists of the invention may cross-react with CD27 from species other than human. Alternatively, the CD27 agonists may be specific for human CD27 and may not exhibit any cross-reactivity with other species. CD27 or any variants and isoforms thereof, may either be isolated from cells or tissues that naturally express them or be recombinantly produced using well-known techniques in the art and/or those described herein. Preferably the CD27 agonists are targeted to human CD27 which has a normal glycosylation pattern.

Genbank^{®} (Accession No. AAH12160.1) reports the amino acid sequence of human CD27 as follows (SEQ ID NO: 29):

1 marphpwwlc vlgtlvglsa tpapkscper hywaqgklcc qmcepgtflv kdcdqhrkaa
61 qcdpcipgvs fspdhhtrph cescrhcnsg llvrnctita naecacrngw qcrdkectec
121 dplpnpslta rssqalsphp qpthlpyvse mleartaghm qtladfrqlp artlsthwpp
181 qrslcssdfi rilvifsgmf lvftlagalf lhqrrkyrsn kgespvepae pcryscpree
241 egstipiqed yrkpepacsp

The term "CD70" (also referred to as "CD70 molecule", "CD27L", "CD27LG", "TNFSF7," "tumor necrosis factor (ligand) superfamily member 7," "CD27 ligand," "CD70 antigen," "surface antigen CD70," "tumor necrosis factor ligand superfamily, member 7," "Ki-24 antigen," and "CD27-L") refers to the ligand for CD27 (see, for example, Bowman MR et al., J. Immunol. 1994 Feb 15;152(4):1756-61). CD70 is a type II transmembrane protein that belongs to the tumor necrosis factor (TNF) ligand family. It is a surface antigen on activated T and B lymphocytes that induces proliferation of co-stimulated T cells, enhances the generation of cytolytic T cells, and contributes to T cell activation. It has also been suggested that CD70 plays a role in regulating B-cell activation, cytotoxic function of natural killer cells, and immunoglobulin synthesis (Hintzen RQ et al., J. Immunol. 1994 Feb 15; 152(4): 1762-73).

Genbank^{®} (Accession No. NP_001243) reports the amino acid sequence of human CD70 as follows (SEQ ID NO: 30):

1 mpeegsgcsv rrrpygcvlr aalvplvagl viclvvciqr faqaqqqlpl eslgwdvael

61 qlnhtgpqqd prlywqggpa lgrsflhgpe ldkgqlrihr dgiymvhiqv tlaicsstta
121 srhhpttlav gicspasrsi sllrlsfhqg ctiasqrltp largdtlctn ltgtllpsrn
181 tdetffgvqw vrp

An exemplary human CD70 extracellular domain (CD70ECD) has the amino acid sequence shown in SEQ ID NO: 31, corresponding to amino acid residues 27-176 of the mature protein.

The term "antibody" as referred to herein includes whole antibodies and any antigen binding fragment (*i.e.,* "antigen-binding portion") or single chain version thereof. An "antibody" refers, in one preferred embodiment, to a glycoprotein comprising at least two heavy (H) chains and two light (L) chains inter-connected by disulfide bonds, or an antigen binding portion thereof. Each heavy chain is comprised of a heavy chain variable region (abbreviated herein as V_{H}) and a heavy chain constant region. The heavy chain constant region is comprised of three domains, CH1, CH2 and CH3. Each light chain is comprised of a light chain variable region (abbreviated herein as V_{L}) and a light chain constant region. The light chain constant region is comprised of one domain, CL. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (*e.g.,* effector cells) and the first component (Clq) of the classical complement system.

The term "antigen-binding fragment" of an antibody (or simply "antibody fragment"), as used herein, refers to one or more fragments or portions of an antibody that retain the ability to specifically bind to an antigen (*e.g*., human CD27). Such "fragments" are, for example between about 8 and about 1500 amino acids in length, suitably between about 8 and about 745 amino acids in length, suitably about 8 to about 300, for example about 8 to about 200 amino acids, or about 10 to about 50 or 100 amino acids in length. It has been shown that the antigen-binding function of an antibody can be performed by fragments of a full-length antibody. Examples of binding fragments encompassed within the term "antigen-binding fragment" of an antibody include (i) a Fab fragment, a monovalent fragment consisting of the V_{L}, V_{H}, CL and CH1 domains; (ii) a F(ab')₂ fragment, a bivalent fragment comprising two Fab fragments linked by a disulfide bridge at the hinge region; (iii) a Fd fragment consisting of the V_{H} and CH1 domains; (iv) a Fv fragment consisting of the V_{L} and V_{H} domains of a single arm of an antibody, (v) a dAb fragment (Ward et al., (1989) Nature 341:544-546), which consists of a V_{H} domain; and (vi) an isolated complementarity determining region (CDR) or (vii) a combination of two or more isolated CDRs which may optionally be joined by a synthetic linker. Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (sFv); see *e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding fragment" of an antibody. These antibody fragments are obtained using conventional techniques known to those with skill in the art, and the fragments are screened for utility in the same manner as are intact antibodies. Antigen-binding portions can be produced by recombinant DNA techniques, or by enzymatic or chemical cleavage of intact immunoglobulins.

The term "monoclonal antibody," as used herein, refers to an antibody that displays a single binding specificity and affinity for a particular epitope. Accordingly, the term "human monoclonal antibody" refers to an antibody which displays a single binding specificity and which has variable and optional constant regions derived from human germline immunoglobulin sequences. In one embodiment, human monoclonal antibodies are produced by a hybridoma that includes a B cell obtained from a transgenic non-human animal, *e.g.,* a transgenic mouse, having a genome comprising a human heavy chain transgene and a light chain transgene fused to an immortalized cell.

The term "recombinant human antibody," as used herein, includes all human antibodies that are prepared, expressed, created or isolated by recombinant means, such as (a) antibodies isolated from an animal (*e.g.,* a mouse) that is transgenic or transchromosomal for human immunoglobulin genes or a hybridoma prepared therefrom, (b) antibodies isolated from a host cell transformed to express the antibody, *e.g*., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies comprise variable and constant regions that utilize particular human germline immunoglobulin sequences are encoded by the germline genes, but include subsequent rearrangements and mutations which occur, for example, during antibody maturation. As known in the art (see, *e.g.,* Lonberg (2005) Nature Biotech. 23(9): 1117-1125), the variable region contains the antigen binding domain, which is encoded by various genes that rearrange to form an antibody specific for a foreign antigen. In addition to rearrangement, the variable region can be further modified by multiple single amino acid changes (referred to as somatic mutation or hypermutation) to increase the affinity of the antibody to the foreign antigen. The constant region will change in further response to an antigen (*i.e.,* isotype switch). Therefore, the rearranged and somatically mutated nucleic acid molecules that encode the light chain and heavy chain immunoglobulin polypeptides in response to an antigen may not have sequence identity with the original nucleic acid molecules, but instead will be substantially identical or similar (*i.e.,* have at least 80% identity).

The term "human antibody" includes antibodies having variable and constant regions (if present) of human germline immunoglobulin sequences. Human antibodies of the invention can include amino acid residues not encoded by human germline immunoglobulin sequences (*e.g.,* mutations introduced by random or site-specific mutagenesis *in vitro* or by somatic mutation *in vivo)* (*see,* Lonberg, N. et al. (1994) Nature 368(6474): 856-859); Lonberg, N. (1994) Handbook of Experimental Pharmacology 113:49-101; Lonberg, N. and Huszar, D. (1995) Intern. Rev. Immunol. Vol. 13: 65-93, and Harding, F. and Lonberg, N. (1995) Ann. N.Y. Acad. Sci 764:536-546). However, the term "human antibody" does not include antibodies in which CDR sequences derived from the germline of another mammalian species, such as a mouse, have been grafted onto human framework sequences (*i.e.,* humanized antibodies).

An "isolated antibody," as used herein, is intended to refer to an antibody which is substantially free of other antibodies having different antigenic specificities (*e.g*., an isolated antibody that specifically binds to human CD27 is substantially free of antibodies that specifically bind antigens other than human CD27). An isolated antibody that specifically binds to an epitope of may, however, have cross-reactivity to other CD27 proteins from different species. However, the antibody preferably always binds to human CD27. In addition, an isolated antibody is typically substantially free of other cellular material and/or chemicals. A combination of "isolated" antibodies having different CD27 specificities may be combined in a well-defined composition.

The term "epitope" or "antigenic determinant" refers to a site on an antigen to which an immunoglobulin or antibody specifically binds. Epitopes can be formed both from contiguous amino acids or noncontiguous amino acids juxtaposed by tertiary folding of a protein. Epitopes formed from contiguous amino acids are typically retained on exposure to denaturing solvents, whereas epitopes formed by tertiary folding are typically lost on treatment with denaturing solvents. An epitope typically includes at least 3, 4, 5, 6, 7, 8, 9, 10, 11, 12, 13, 14 or 15 amino acids in a unique spatial conformation. Methods for determining what epitopes are bound by a given antibody (*i.e.,* epitope mapping) are well known in the art and include, for example, immunoblotting and immunoprecipitation assays, wherein overlapping or contiguous peptides from CD27 are tested for reactivity with the given anti-CD27 antibody. Methods of determining spatial conformation of epitopes include techniques in the art and those described herein, for example, x-ray crystallography and 2-dimensional nuclear magnetic resonance (see, *e.g.,* Epitope Mapping Protocols in Methods in Molecular Biology, Vol. 66, G. E. Morris, Ed. (1996)).

The term "antibody that binds the same epitope" as another antibody is intended to encompass antibodies that interact with, i.e., bind to, the same structural region on human CD27 as a reference anti-CD27 antibody. The "same epitope" to which the antibodies bind may be a linear epitope or a conformational epitope formed by tertiary folding of the antigen.

The term "competing antibody" refers to an antibody that competes for binding to human CD27 with a reference anti-CD27 antibody, i.e., competitively inhibits binding of the reference anti-CD27 antibody to CD27. A "competing antibody" may bind the same epitope on CD27 as the reference anti-CD27 antibody, may bind to an overlapping epitope or may sterically hinder the binding of the reference anti-CD27 antibody to CD27.

Antibodies that recognize the same epitope or compete for binding can be identified using routine techniques. Such techniques include, for example, an immunoassay, which shows the ability of one antibody to block the binding of another antibody to a target antigen, *i.e.,* a competitive binding assay. Competitive binding is determined in an assay in which the immunoglobulin under test inhibits specific binding of a reference antibody to a common antigen, such as CD27. Numerous types of competitive binding assays are known, for example: solid phase direct or indirect radioimmunoassay (RIA), solid phase direct or indirect enzyme immunoassay (EIA), sandwich competition assay (see Stahli et al., Methods in Enzymology 9:242 (1983)); solid phase direct biotin-avidin EIA (see Kirkland et al., J. Immunol. 137:3614 (1986)); solid phase direct labeled assay, solid phase direct labeled sandwich assay (see Harlow and Lane, Antibodies: A Laboratory Manual, Cold Spring Harbor Press (1988)); solid phase direct label RIA using 1-125 label (see Morel et al., Mol. Immunol. 25(1):7 (1988)); solid phase direct biotin-avidin EIA (Cheung et al., Virology 176:546 (1990)); and direct labeled RIA. (Moldenhauer et al., Scand. J. Immunol. 32:77 (1990)). Typically, such an assay involves the use of purified antigen bound to a solid surface or cells bearing either of these, an unlabeled test immunoglobulin and a labeled reference immunoglobulin. Competitive inhibition is measured by determining the amount of label bound to the solid surface or cells in the presence of the test immunoglobulin. Usually the test immunoglobulin is present in excess. Usually, when a competing antibody is present in excess, it will inhibit specific binding of a reference antibody to a common antigen by at least 50-55%, 55-60%, 60-65%, 65-70% 70-75% or more.

Other techniques include, for example, epitope mapping methods, such as, x-ray analyses of crystals of antigen: antibody complexes which provides atomic resolution of the epitope. Other methods monitor the binding of the antibody to antigen fragments or mutated variations of the antigen where loss of binding due to a modification of an amino acid residue within the antigen sequence is often considered an indication of an epitope component. In addition, computational combinatorial methods for epitope mapping can also be used. These methods rely on the ability of the antibody of interest to affinity isolate specific short peptides from combinatorial phage display peptide libraries. The peptides are then regarded as leads for the definition of the epitope corresponding to the antibody used to screen the peptide library. For epitope mapping, computational algorithms have also been developed which have been shown to map conformational discontinuous epitopes.

As used herein, the terms "specific binding," "selective binding," "selectively binds," and "specifically binds," refer to antibody binding to an epitope on a predetermined antigen. Typically, the antibody binds with an equilibrium dissociation constant (K_{D}) of approximately less than 10⁻⁷ M, such as approximately less than 10 ⁻⁸ M, 10⁻⁹ M or 10⁻¹⁰ M or even lower when determined by surface plasmon resonance (SPR) technology in a BIACORE 2000 instrument using recombinant human CD27 as the analyte and the antibody as the ligand and binds to the predetermined antigen with an affinity that is at least two-fold greater than its affinity for binding to a non-specific antigen (*e.g*., BSA, casein) other than the predetermined antigen or a closely-related antigen. The phrases "an antibody recognizing an antigen" and "an antibody specific for an antigen" are used interchangeably herein with the term "an antibody which binds specifically to an antigen."

As used herein, "isotype" refers to the antibody class (*e.g.,* IgM or IgGl) that is encoded by heavy chain constant region genes. In one embodiment, an anti-CD27 antibody used in a CD27 agonist of the invention is of the IgG1 isotype. In another embodiment, an anti-CD27 antibody used in a CD27 agonist of the invention is of the IgG2 isotype.

The term "binds to immobilized CD27," refers to the ability of a CD27 agonist of the invention to bind to CD27, for example, expressed on the surface of a cell or which is attached to a solid support.

The term "cross-reacts," as used herein, refers to the ability of a CD27 agonist of the invention to bind to CD27 from a different species. For example, a CD27 agonist of the invention that binds human CD27 may also bind another species of CD27. As used herein, cross-reactivity is measured by detecting a specific reactivity with purified antigen in binding assays (*e.g.*, SPR, ELISA) or binding to, or otherwise functionally interacting with, cells physiologically expressing CD27. Methods for determining cross-reactivity include standard binding assays as described herein, for example, by Biacore^{™} surface plasmon resonance (SPR) analysis using a Biacore^{™} 2000 SPR instrument (Biacore AB, Uppsala, Sweden), or flow cytometric techniques.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory is naturally-occurring.

The term "nucleic acid molecule," as used herein, is intended to include DNA molecules and RNA molecules. A nucleic acid molecule may be single-stranded or double-stranded, but preferably is double-stranded DNA.

The term "isolated nucleic acid molecule," as used herein in reference to nucleic acids encoding antibodies or antibody portions (*e.g*., V_{H}, V_{L}, CDR3) that bind to CD27, is intended to refer to a nucleic acid molecule in which the nucleotide sequences encoding the antibody or antibody portion are free of other nucleotide sequences encoding antibodies or antibody portions that bind antigens other than CD27, which other sequences may naturally flank the nucleic acid in human genomic DNA.

"Conservative sequence modifications" of any of the sequences set forth in SEQ ID NOs: 1-75, *i.e.,* nucleotide and amino acid sequence modifications, are modifications which do not abrogate the binding of the VH and VL sequences encoded by the nucleotide sequence or containing the amino acid sequence, to the antigen. Such conservative sequence modifications include conservative nucleotide and amino acid substitutions, as well as, nucleotide and amino acid additions and deletions. For example, modifications can be introduced into SEQ ID NOs: 1-75 by standard techniques known in the art, such as site-directed mutagenesis and PCR-mediated mutagenesis. Conservative amino acid substitutions include ones in which the amino acid residue is replaced with an amino acid residue having a similar side chain. Families of amino acid residues having similar side chains have been defined in the art. These families include amino acids with basic side chains (*e.g*., lysine, arginine, histidine), acidic side chains (*e.g*., aspartic acid, glutamic acid), uncharged polar side chains (*e.g*., glycine, asparagine, glutamine, serine, threonine, tyrosine, cysteine, tryptophan), nonpolar side chains (*e.g*., alanine, valine, leucine, isoleucine, proline, phenylalanine, methionine), beta-branched side chains (*e.g*., threonine, valine, isoleucine) and aromatic side chains (*e.g*., tyrosine, phenylalanine, tryptophan, histidine). Thus, a predicted nonessential amino acid residue in an anti-CD27 antibody is preferably replaced with another amino acid residue from the same side chain family. Methods of identifying nucleotide and amino acid conservative substitutions which do not eliminate antigen binding are well-known in the art (see, *e.g.,* Brummell et al., Biochem. 32:1180-1187 (1993); Kobayashi et al. Protein Eng. 12(10):879-884 (1999); and Burks et al. Proc. Natl. Acad. Sci. USA 94:412-417 (1997))

Alternatively, mutations can be introduced randomly along all or part of an anti-CD27 antibody coding sequence, such as by saturation mutagenesis, and the resulting modified anti-CD27 antibodies can be screened for binding activity.

For nucleic acids, the term "substantial homology" indicates that two nucleic acids, or designated sequences thereof, when optimally aligned and compared, are identical, with appropriate nucleotide insertions or deletions, in at least about 80% of the nucleotides, usually at least about 90% to 95%, and more preferably at least about 98% to 99.5% of the nucleotides. Alternatively, substantial homology exists when the segments will hybridize under selective hybridization conditions, to the complement of the strand.

For amino acids, the term "substantial homology" indicates that two amino acid sequences, or designated sequences thereof, when optimally aligned and compared, are identical, with appropriate amino acid insertions or deletions, in at least about 80% of the amino acids, usually at least about 90% to 95%, and more preferably at least about 98% to 99% or 99.5% of the amino acids.

The percent identity between two sequences is a function of the number of identical positions shared by the sequences (*i.e.,* % homology = # of identical positions/total # of positions x 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two nucleotide sequences can be determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. The percent identity between two nucleotide or amino acid sequences can also be determined using the algorithm of E. Meyers and W. Miller (CABIOS, 4:11-17 (1989)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. (48):444-453 (1970)) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

Nucleic acid and protein sequences can further be used as a "query sequence" to perform a search against public databases to, for example, identify related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to the nucleic acid molecules of the invention. BLAST protein searches can be performed with the XBLAST program, score = 50, wordlength = 3 to obtain amino acid sequences homologous to the protein molecules of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (*e.g*., XBLAST and NBLAST) can be used. See http://www.ncbi.nlm.nih.gov.

The nucleic acids may be present in whole cells, in a cell lysate, or in a partially purified or substantially pure form. A nucleic acid is "isolated" or "rendered substantially pure" when purified away from other cellular components or other contaminants, *e.g*., other cellular nucleic acids or proteins, by standard techniques, including alkaline/SDS treatment, CsCl banding, column chromatography, agarose gel electrophoresis and others well known in the art. *See,* F. Ausubel, et al., ed. Current Protocols in Molecular Biology, Greene Publishing and Wiley Interscience, New York (1987).

Nucleic acid compositions, while often in a native sequence (except for modified restriction sites and the like), from either cDNA, genomic or mixtures thereof may be mutated, in accordance with standard techniques to provide gene sequences. For coding sequences, these mutations, may affect amino acid sequence as desired. In particular, DNA sequences substantially homologous to or derived from native V, D, J, constant, switches and other such sequences described herein are contemplated (where "derived" indicates that a sequence is identical or modified from another sequence).

A nucleic acid is "operably linked" or "operatively linked" when it is placed into a functional relationship with another nucleic acid sequence. For instance, a promoter or enhancer is operably linked to a coding sequence if it affects the transcription of the sequence. With respect to transcription regulatory sequences, operably linked means that the DNA sequences being linked are contiguous and, where necessary to join two protein coding regions, contiguous and in reading frame. For switch sequences, operably linked indicates that the sequences are capable of effecting switch recombination.

The term "vector," as used herein, is intended to refer to a nucleic acid molecule capable of transporting another nucleic acid to which it has been linked. One type of vector is a "plasmid," which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g*., bacterial vectors having a bacterial origin of replication and episomal mammalian vectors). Other vectors (*e.g*., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, other forms of expression vectors, such as viral vectors (*e.g*., replication defective retroviruses, adenoviruses and adeno-associated viruses), serve equivalent functions.

The term "recombinant host cell" (or simply "host cell"), as used herein, is intended to refer to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

As used herein, the term "antigen" refers to any natural or synthetic immunogenic substance, such as a protein, peptide, or hapten. Suitable antigens for use in the present invention (*e.g*., in a vaccine in combination with a CD27 agonist of the invention) include, for example, infectious disease antigens and tumor antigens, against which protective or therapeutic immune responses are desired, *e.g*., antigens expressed by a tumor cell or a pathogenic organism or infectious disease antigens. For example, suitable antigens include tumor-associated antigens for the prevention or treatment of cancers. Examples of tumor-associated antigens (TAAs) include, but are not limited to, sequences comprising all or part of the sequences of EGFR, EGFRvIII, gp100 or Pmel17, HER2/neu, mesothelin, CEA, MARTI, MAGE-A1, MAGE-A2, MAGE-A3, MAGE-A4, MUC-1, GPNMB, HMW-MAA, TIM1, ROR1, CD19 and germ cell derived tumor antigens. Alternatively, more than one antigen can be included within antigen-antibody constructs. For example, a MAGE antigen can be combined with other antigens such as MART1 and gp100 along with adjuvants such as GM-CSF or IL-12, and linked to an anti-APC antibody.

Other suitable antigens include viral antigens for the prevention or treatment of viral diseases. Examples of viral antigens include, but are not limited to, HIV-1 env, HBsAg, HPV, FAS, HSV-1, HSV-2, p17, ORF2 and ORF3 antigens. Examples of bacterial antigens include, but are not limited to, *Toxoplasma gondii* or *Treponema pallidum.* The bacterial antigens can be in the treatment or prevention of various bacterial diseases such as Anthrax, Botulism, Tetanus, Chlamydia, Cholera, Diphtheria, Lyme Disease, Syphilis and Tuberculosis. Other suitable antigens from infectious disease pathogens, such as viruses, bacteria, parasites and fungi are disclosed below.

Sequences of the foregoing antigens are well known in the art. For example, an example of a MAGE-3 cDNA sequence is provided in US 6,235,525 (Ludwig Institute for Cancer Research); examples of NY-ESO-1 nucleic acid and protein sequences are provided in US 5,804,381 and US 6,069,233 (Ludwig Institute for Cancer Research); examples of MART1 nucleic acid and protein sequences are provided in US 5,620,886 and US 5,854,203 (Ludwig Institute for Cancer Research); an example of an amino acid sequence for the HER-2/neu protein is available at GENBANK^{®} Accession No. AAA58637; and a nucleotide sequence (mRNA) for human carcinoembryonic antigen-like 1 (CEA-1) is available at GENBANK^{®} Accession No. NM _020219.

An HPV antigen that may be used in the compositions and the methods of the invention may include, for example an HPV-16 antigen, an HPV- 18 antigen, an HPV-31 antigen, an HPV-33 antigen and/or an HPV-35 antigen; and is suitably an HPV-16 antigen and/or HPV-18 antigen. A genome of HPV-16 is described in Virology, 145:181- 185 (1985) and DNA sequences encoding HPV-18 are described in US Patent No. 5,840,306. HPV-16 antigens (e.g., seroreactive regions of the E1 and/or E2 proteins of HPV-16) are described in US Patent No. 6,531,127, and HPV-18 antigens (e.g., seroreactive regions of the L1 and/or L2 proteins of HPV-18) are described in US Patent No. 5,840,306. Similarly, a complete genome for HBV is available at GENBANK^{®} Accession No. NC_003977. The genome of HCV is described in European Patent Application No. 318 216. PCT/US90/01348 discloses sequence information of clones of the HCV genome, amino acid sequences of HCV viral proteins and methods of making and using such compositions for HCV vaccines comprising HCV proteins and peptides derived there from.

Antigenic peptides of proteins (*i.e.,* those containing T cell epitopes) can be identified in a variety of manners well known in the art. For example, T cell epitopes can be predicted by analyzing the sequence of the protein using web-based predictive algorithms (BIMAS & SYFPEITHI) to generate potential MHC class I and II- binding peptides that match an internal database of 10,000 well characterized MHC binding peptides previously defined by CTLs. High scoring peptides can be ranked and selected as "interesting" on the basis of high affinity to a given MHC molecule.

Another method for identifying antigenic peptides containing T cell epitopes is by dividing the protein into non-overlapping peptides of desired length or overlapping peptides of desired lengths which can be produced recombinantly, synthetically, or in certain limited situations, by chemical cleavage of the protein and tested for immunogenic properties, *e.g*., eliciting a T cell response (*i.e.,* proliferation or lymphokine secretion).

In order to determine precise T cell epitopes of the protein by, for example, fine mapping techniques, a peptide having T cell stimulating activity and thus comprising at least one T cell epitope, as determined by T cell biology techniques, can be modified by addition or deletion of amino acid residues at either the amino or carboxy terminus of the peptide and tested to determine a change in T cell reactivity to the modified peptide. If two or more peptides which share an area of overlap in the native protein sequence are found to have human T cell stimulating activity, as determined by T cell biology techniques, additional peptides can be produced comprising all or a portion of such peptides and these additional peptides can be tested by a similar procedure. Following this technique, peptides are selected and produced recombinantly or synthetically. Peptides are selected based on various factors, including the strength of the T cell response to the peptide (*e.g*., stimulation index). The physical and chemical properties of these selected peptides (*e.g*., solubility, stability) can then be examined to determine whether the peptides are suitable for use in therapeutic compositions or whether the peptides require modification.

As used herein, the term "immunostimulatory agent" includes but is not limited to compounds capable of stimulating antigen presenting cells (APCs), such as dendritic cells (DCs) and macrophages. For example, suitable immunostimulatory agents for use in the present invention are capable of stimulating APCs, so that the maturation process of the APCs is accelerated, the proliferation of APCs is increased, and/or the recruitment or release of co-stimulatory molecules (*e.g*., CD80, CD86, ICAM-1, MHC molecules and CCR7) and pro-inflammatory cytokines (*e.g*., IL-1β, IL-6, IL-12, IL-15, and IFN-γ) is upregulated. Suitable immunostimulatory agents are also capable of increasing T cell proliferation. Such immunostimulatory agents include, but are not be limited to, CD40 ligand; FLT 3 ligand; cytokines, such as IFN-α, IFN-β, IFN-γ and IL-2; colony-stimulating factors, such as G-CSF (granulocyte colony-stimulating factor) and GM-CSF (granulocyte-macrophage colony-stimulating factor); an anti-CTLA-4 antibody, anti-PD1 antibody, anti-41BB antibody, or anti-OX-40 antibody; LPS (endotoxin); ssRNA; dsRNA; Bacille Calmette-Guerin (BCG); Levamisole hydrochloride; and intravenous immune globulins. An immunostimulatory agant may be a Toll-like Receptor (TLR) agonist. For example the immunostimulatory agent may be a TLR3 agonist such as double-stranded inosine:cytosine polynucleotide (Poly I:C, for example available as AmpligenTM from Hemispherx Bipharma, PA, US or Poly IC:LC from Oncovir) or Poly A:U; a TLR4 agonist such as monophosphoryl lipid A (MPL) or RC-529 (for example as available from GSK, UK); a TLR5 agonist such as flagellin; a TLR7 or TLR8 agonist such as an imidazoquinoline TLR7 or TLR 8 agonist, for example imiquimod (eg AldaraTM) or resiquimod and related imidazoquinoline agents (for example as available from 3M Corporation); or a TLR 9 agonist such as a deoxynucleotide with unmethylated CpG motifs (so-called "CpGs", for example as available from Coley Pharmaceutical). A preferred immunostimulatory agent is a TLR3 agonist, preferably Poly I:C. Such immunostimulatory agents may be administered simultaneously, separately or sequentially with the CD27 agonists of the present invention and may also be physically linked to the CD27 agonists.

As used herein, the term "linked" refers to the association of two or more molecules. The linkage can be covalent or non-covalent. The linkage also can be genetic (*i.e.,* recombinantly fused). Such linkages can be achieved using a wide variety of art recognized techniques, such as chemical conjugation and recombinant protein production.

As used herein, the term "T cell-mediated response" refers to any response mediated by T cells, including effector T cells (*e.g.,* CD8⁺ cells) and helper T cells (*e.g.,* CD4⁺ cells). T cell mediated responses include, for example, T cell cytotoxicity and proliferation.

As used herein, the terms "inhibits" or "blocks" (*e.g*., referring to inhibition/blocking of binding of CD70 to CD27 on cells) are used interchangeably and encompass both partial and complete inhibition/blocking. The inhibition/blocking of CD70 preferably reduces or alters the normal level or type of activity that occurs when CD70 binding occurs without inhibition or blocking. Inhibition and blocking are also intended to include any measurable decrease in the binding affinity of CD70 when in contact with an anti-CD27 antibody as compared to CD70 not in contact with an anti-CD27 antibody, *e.g*., inhibits binding of CD70 by at least about 10%, 15%, 20%, 25%, 30%, 35%, 40%, 45%, 50%, 55%, 60%, 65%, 70%, 75% , 80%, 85%, 90%, 95%, 96%, 97%, 98%, 99%, or 100%. In a preferred embodiment, the anti-CD27 antibody inhibits binding of CD70 by at least about 70%. In another embodiment, the anti-CD27 antibody inhibits binding of CD70 by at least 80%

As used herein, the term "inhibits growth" (*e.g*., referring to cells) is intended to include any measurable decrease in the growth of a cell, *e.g.,* the inhibition of growth of a cell by at least about 10%, 20%, 30%, 40%, 50%, 60%, 70%, 80%, 90%, 99%, or 100%.

The terms "inducing an immune response" and "enhancing an immune response" are used interchangeably and refer the stimulation of an immune response (*i.e.,* either passive or adaptive) to a particular antigen.

The terms "treat," "treating," and "treatment," as used herein, refer to therapeutic or preventative measures described herein. The methods of "treatment" employ administration to a subject, in need of such treatment, a human antibody of the present invention, for example, a subject in need of an enhanced immune response against a particular antigen or a subject who ultimately may acquire such a disorder, in order to prevent, cure, delay, reduce the severity of, or ameliorate one or more symptoms of the disorder or recurring disorder, or in order to prolong the survival of a subject beyond that expected in the absence of such treatment.

The term "effective dose" or "effective dosage" is defined as an amount sufficient to achieve or at least partially achieve the desired effect. The term "therapeutically effective dose" is defined as an amount sufficient to cure or at least partially arrest the disease and its complications in a patient already suffering from the disease. Amounts effective for this use will depend upon the severity of the disorder being treated and the general state of the patient's own immune system.

The term "patient" includes human and other mammalian subjects that receive either prophylactic or therapeutic treatment.

As used herein, the term "subject" includes any human or non-human animal. For example, the methods and compositions of the present invention can be used to treat a subject with an immune disorder. The term "non-human animal" includes all vertebrates, *e.g*., mammals and non-mammals, such as non-human primates, sheep, dog, cow, chickens, amphibians, reptiles, *etc.*

### II. CD27 Hyper-Crosslinking Agents

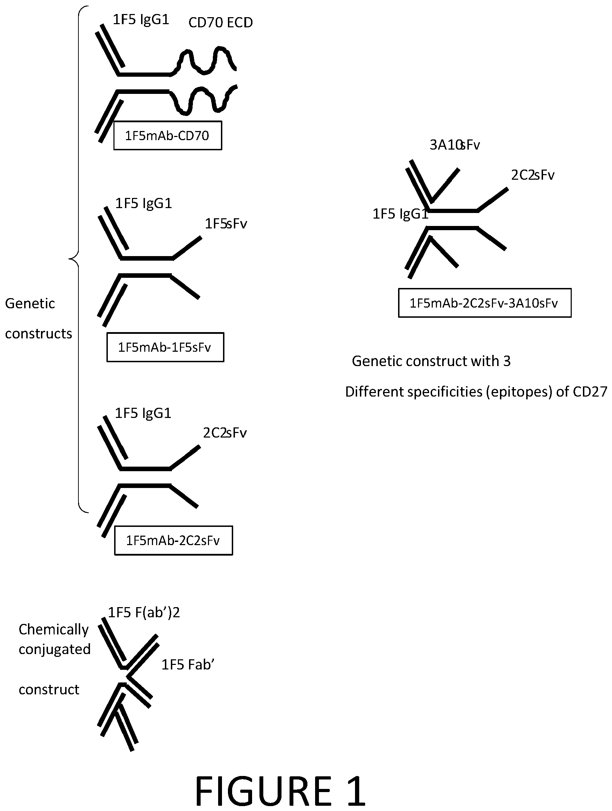

CD27 agonists that are referred to herein as CD27 crosslinking or hyper-crosslinking agents are single molecules that bind three or more CD27 molecules simultaneously. To achieve this, additional CD27 binding domains are chemically or genetically added to an anti-CD27 antibody, or antigen-binding fragment thereof. The added CD27 binding domains can be from the same anti-CD27 antibody, from a different anti-CD27 antibody that binds the same CD27 epitope, from a different anti-CD27 antibody that binds a different CD27 epitope or from a CD27 ligand (*e.g.,* CD70). Non-limiting examples of CD27 hyper-crosslinking agent are illustrated in Figure 1.

These constructs can include a whole anti-CD27 antibody and retain FcR interaction or that domain can be eliminated or mutated. The additional CD27 binding domains can be as little as one, or added in many multiples. Finally, the CD27 binding domains can come from a single source (CD70 or specific anti-CD27 Ab), or from multiple different sources. One such CD27 agonist comprises a first component that is an anti-CD27 antibody, or antigen-binding fragment thereof, linked to a second component comprising one or more cross-linking moieties, wherein
a. at least one of the cross-linking moieties does not bind to an Fc receptor; and
b. the CD27 agonist stimulates T cell activity without the need for Fc receptor interaction;
wherein the second component binds CD27.

The second component may comprise one or more anti-CD27 antibodies, or antigen-binding fragments thereof. The second component may comprise the same anti-CD27 antibody that is in the first component. The second component may comprise one or more different anti-CD27 antibodies than is in the first component. The one or more different anti-CD27 antibodies can bind to the same CD27 epitope as does the first component or, alternatively, the one or more different anti-CD27 antibodies can bind to a different CD27 epitope as does the first component. The second component may comprise an sFv fragment or one or more Fab fragments.

The second component may comprise a CD27 ligand, such as CD70 or a CD70 extracellular domain.

A CD27 agonist comprising a first component that is an anti-CD27 antibody, or antigen-binding fragment thereof, linked to a second component comprising one or more cross-linking moieties having a binding specificity that is the same as the first component or a CD27 agonist comprising a first component that is an anti-CD27 antibody, or antigen-binding fragment thereof, linked to a second component comprising a CD27 ligand may stimulate T cell activity without the need for Fc receptor interaction.

The CD27 agonists may or may not contain any functional Fc receptor-binding domains. For example, the CD27 agonist may comprise a second component comprising one or more cross-linking moieties in which none of the cross-linking moieties contain a functional Fc receptor-binding domain. The CD27 agonist may comprise a second component comprising one or more cross-linking moieties in which one or more of the cross-linking moieties does not contain a functional Fc receptor-binding domain and one or more of the cross-linking moieties does contain a functional Fc receptor-binding domain.

The CD27 agonist may be one that does not significantly activate regulatory T cells (Tregs) expressing CD27.

A CD27 agonist that is a CD27 hyper-crosslinking agent may be a fusion protein, which can be prepared by recombinant means, as described in further detail in subsection V below and in Example 2. A CD27 agonist that is a CD27 hyper-crosslinking agent may be a chemical conjugate, which can be prepared by chemical reaction using a cross-linking agent, as described in further detail in subsection V below and in Reference Example 1.

Specific CD27 agonists that are CD27 hyper-crosslinking agents, and specific first and second components that can be used therein, are described in further detail in subsection IV below.

### III. CD27 Multi-Specific Agents

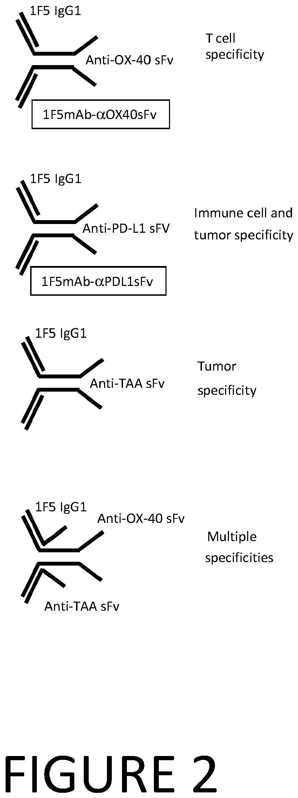

CD27 agonists that are referred to herein as multi-specific agents are molecules that bind to CD27 and to at least one additional molecule other than FcR. To achieve this, additional ligand or Ab binding domains that bind a target other than CD27 or FcR are chemically or genetically added to an anti-CD27 antibody, or antigen-binding fragment thereof. The added binding domains can be specific for another molecule on T cells, or can be specific for molecules expressed, for example, by other immune cells, by tumor cells or by stromal cells. Non-limiting examples of multi-specific agents are illustrated in Figure 2.

These constructs can include a whole anti-CD27 antibody and retain FcR interaction or that domain can be eliminated or mutated. The additional binding domains can be as little as one, or added in many multiples. Finally, these binding domains can include a single specificity, or include multiple different specificities.

A CD27 agonist comprising a first component that is an anti-CD27 antibody, or antigen-binding fragment thereof, may be linked to a second component comprising one or more cross-linking moieties, wherein
a. at least one of the cross-linking moieties does not bind to an Fc receptor; and
b. the CD27 agonist stimulates T cell activity without the need for Fc receptor interaction;
wherein the second component comprises one or more cross-linking moieties that bind one or more molecules other than CD27 or FcR.

The second component may comprise two or more cross-linking moieties that bind at least two different molecules other than CD27.

A CD27 agonist comprising a first component that is an anti-CD27 antibody, or antigen-binding fragment thereof, may be linked to a second component comprising two or more cross-linking moieties, wherein
a. the two or more cross-linking moieties do not bind to an Fc receptor;
b. each of the two or more cross-linking molecules bind different molecules; and
c. the CD27 agonist stimulates T cell activity without the need for Fc receptor interaction.

In the multi-specific agents, the second component may comprise one or more antibodies, or antigen binding fragments thereof. The second component may comprise an sFv fragment. The second component may comprise one or more Fab fragments. The second component may comprise one or more ligands that bind the target molecule (other than CD27 or FcR), such as an extracellular domain of a ligand that binds the target molecule.

The second component may comprise one or more cross-linking moieties that bind a molecule on T cells. Preferred molecules on T cells that can be the target of the second component include OX-40, 41BB, CD28, ICOS, CD40L, GITR, TIM1, CD30, HVEM, LIGHT, SLAM, DR3, CD2, CD226, PD-1, CTLA4, LAG3, CD160, BTLA, VISTA, LAIR1, TIM3, 2B4 and TIGIT. The second component may comprise one or more cross-linking moieties that bind a molecule on T cells, with the proviso that the molecule is not CD3, CD40, CD25, CD137, CD154, an Fc receptor or CD27. The second component may bind to a positive costimulatory receptor on T-cells (examples of which include but are not limited to OX-40, CD28, GITR and ICOS). The second component may bind to a negative costimulatory receptor on T-cells or a ligand thereof (examples of which include but are not limited to PD-1, PD-L1, PD-L2, CTLA4, BTLA and VISTA).

The second component may comprise one or more cross-linking moieties that bind a molecule on an immune cell, such as a B lymphocyte or an NK cell. The second component may comprise one or more cross-linking moieties that bind a molecule on B lymphocytes, with the proviso that the molecule is not CD19 or CD20. The second component may comprise one or more cross-linking moieties that bind a molecule on NK cells, with the proviso that the molecule is not CD56.

The second component may comprise one or more cross-linking moieties that bind a molecule on a stromal cell.

The second component may comprise one or more cross-linking moieties that bind a molecule on a tumor cell. Preferred molecules on tumor cells that can be the target of the second component include tumor associated antigens (TAAs) such as EGFR, EGFRvIII, gp100 or Pmel17, HER2/neu, mesothelin, CEA, MARTI, MAGE-A1, MAGE-A2, MAGE-A3, MAGE-A4, MUC-1, GPNMB, HMW-MAA, TIM1, ROR and CD19.

The second component may comprise one or more cross-linking moieties that bind a molecule both on immune cells and on tumor cells. Preferred molecules both on immune cells and tumor cells that can be the target of the second component include PD-L1, VISTA, B7H3 and B7H4.

The invention pertains to a CD27 agonist comprising a first component that is an anti-CD27 antibody, or antigen-binding fragment thereof, linked to a second component that is an anti-PD-L1 antibody, or antigen-binding fragment thereof. In one embodiment, the second component is an anti-PD-Ll scFv. In one embodiment, the first component is an anti-CD27 antibody and the second component is an anti-PD-Ll scFv linked to the C-terminus of the heavy chain of the anti-CD27 antibody. In one embodiment, (i) the anti-CD27 antibody comprises a VH CDR3 shown in SEQ ID NO: 19 and a VL CDR3 shown in SEQ ID NO: 22 and (ii) the anti-PD-Ll scFv comprises a VH CDR3 shown in SEQ ID NO: 60 and a VL CDR3 shown in SEQ ID NO: 63. Sequences substantially homologous (e.g. at least 80%, 90%, 95%, 98% or 99% identical to the aforementioned sequences) are also encompassed. In another embodiment, (i) the anti-CD27 antibody comprises VH CDR1, CDR2 and CDR3 shown in SEQ ID NOs: 17, 18 and 19, respectively, and VL CDR1, CDR2 and CDR3 shown in SEQ ID NOs: 20, 21 and 22, respectively, and (ii) the anti-PD-Ll scFv comprises VH CDR1, CDR2 and CDR3 shown in SEQ ID NOs: 58, 59 and 60, respectively, and VL CDR1, CDR2 and CDR3 shown in SEQ ID NOs: 61, 62 and 63, respectively. In another embodiment, (i) the anti-CD27 antibody comprises VH and VL chains comprising the amino acid sequences shown in SEQ ID NOs: 1 and 2, respectively; and (ii) the anti-PD-Ll scFv comprises VH and VL chains comprising the amino acid sequences shown in SEQ ID NOs: 32 and 33, respectively. Sequences substantially homologous (e.g. at least 80%, 90%, 95%, 98% or 99% identical to the aforementioned sequences) are also encompassed. In another embodiment, the CD27 agonist comprising an anti-CD27 antibody and an anti-PD-Ll scFv comprises a heavy chain comprising the amino acid sequence shown in SEQ ID NO: 66 and a light chain comprising the amino acid sequence shown in SEQ ID NO: 67. Sequences substantially homologous (e.g. at least 80%, 90%, 95%, 98% or 99% identical to the aforementioned sequences) are also encompassed. In another embodiment, the heavy chain is encoded by the nucleotide sequence shown in SEQ ID NO: 64 and the light chain is encoded by the nucleotide sequence shown in SEQ ID NO: 65. Sequences substantially homologous (e.g. at least 80%, 90%, 95%, 98% or 99% identical to the aforementioned sequences) are also encompassed.

The CD27 agonists can contain no functional Fc receptor-binding domains, or can contain at least one functional Fc receptor-binding domain. For example, the CD27 agonist may comprise a second component comprising one or more cross-linking moieties in which none of the cross-linking moieties contain a functional Fc receptor-binding domain. In another example, the CD27 agonist comprises a second component comprising one or more cross-linking moieties in which one or more of the cross-linking moieties does not contain a functional Fc receptor-binding domain and one or more of the cross-linking moieties does contain a functional Fc receptor-binding domain. The second component may comprise two or more cross-linking moieties wherein at least two of the cross-linking moieties do not bind to an Fc receptor; the second component comprises three or more cross-linking moieties wherein at least three of the cross-linking moieties do not bind to an Fc receptor; the second component comprises four or more cross-linking moieties wherein at least four of the cross-linking moieties do not bind to an Fc receptor; the second component comprises five or more cross-linking moieties wherein at least five of the cross-linking moieties do not bind to an Fc receptor; the second component comprises seven or more cross-linking moieties wherein at least seven of the cross-linking moieties do not bind to an Fc receptor; the second component comprises ten or more cross-linking moieties wherein at least ten of the cross-linking moieties do not bind to an Fc receptor; the second component comprises twenty or more cross-linking moieties wherein at least twenty of the cross-linking moieties do not bind to an Fc receptor; the second component comprises between three and twenty cross-linking moieties wherein at least three of the cross-linking moieties do not bind to an Fc receptor; the second component comprises between four and fifteen cross-linking moieties wherein at least four of the cross-linking moieties do not bind to an Fc receptor; or the second component comprises between five and ten cross-linking moieties wherein at least five of the cross-linking moieties do not bind to an Fc receptor.

The CD27 agonist may be one that does not significantly activate regulatory T cells (Tregs) expressing CD27.

A CD27 agonist that is a multi-specific agent is a fusion protein, can be prepared by recombinant means, as described in further detail in subsection V below and in Example 2. A CD27 agonist that is a multi-specific agent may be a chemical conjugate, which can be prepared by chemical reaction using a cross-linking agent, as described in further detail in subsection V below and in Reference Example 1.

Specific CD27 agonists that are multi-specific agents, and specific first and second components that can be used therein, are described in further detail in subsection IV below.

### IV. Specific CD27 Agonists

In the CD27 agonists, the first component is an anti-CD27 antibody, or antigen-binding fragment thereof. Furthermore, in the CD27 agonists that are CD27 hyper-crosslinking agents, the second component may also comprise one or more anti-CD27 antibodies, or antigen-binding fragments thereof. Preferred anti-CD27 antibodies for use in the agonists are human anti-human CD27 monoclonal antibodies, such as those described in U.S. Patent Publications 20110274685 and 20120213771. Alternatively, non-human monoclonal antibodies (e.g., murine antibodies), as well as chimeric or humanized anti-CD27 antibodies also are encompassed for use in the agonists.

Preferred human anti-human CD27 antibodies for use in the agonists include the antibodies disclosed in U.S. Patent Publications 20110274685 and 20120213771 that are referred to as follows and having VH and VL sequences as follows:
1F5: having VH and VL sequences shown in SEQ ID NOs: 1 and 2, respectively;
2C2: having VH and VL sequences shown in SEQ ID NOs: 3 and 4, respectively;
3H12: having VH and VL sequences shown in SEQ ID NOs: 5 and 6, respectively;
2G9: having VH and VL sequences shown in SEQ ID NOs: 7 and 8, respectively;
1H8: having VH and VL sequences shown in SEQ ID NOs: 9 and 10, respectively;
3A10: having VH and VL sequences shown in SEQ ID NOs: 11 and 12, respectively;
3H8: having VH and VL sequences shown in SEQ ID NOs: 13 and 14, respectively; and
1G5: having VH and VL sequences shown in SEQ ID NOs: 15 and 16, respectively.

Sequences substantially homologous (e.g. at least 80%, 90%, 95%, 98% or 99% identical to the aforementioned sequences) are also encompassed.

Anti-CD27 antibodies that compete for binding with any of the foregoing antibodies, or that bind the same epitope as any of the foregoing antibodies are also suitable.

A particularly preferred human anti-human CD27 antibody for use in the agonist is 1F5, having VH and VL sequences shown in SEQ ID NOs: 1 and 2, respectively. Furthermore, another preferred antibody for use in the agonist is a recombinant anti-CD27 antibody comprising the heavy chain CDR1, CDR2 and CDR3 sequences and light chain CDR1, CDR2 and CDR3 sequences of 1F5, which are shown in SEQ ID NOs: 17, 18, 19 (heavy chain CDR1, CDR2 and CDR3 respectively) and SEQ ID NOs: 20, 21, 22 (heavy chain CDR1, CDR2 and CDR3 respectively). Preferably, the 1F5 antibody (having VH and VL sequences shown in SEQ ID NOs: 1 and 2, respectively) or an antibody comprising the 1F5 VH and VL CDRs (having sequences shown in SEQ ID NOs: 17-22) is used as the first component of the CD27 agonist. Additionally or alternatively, the 1F5 antibody, or an antibody comprising the 1F5 CDRs, can be used as the second component in CD27 hyper-crosslinking agents, in which the second component is one or more anti-CD27 antibodies, or antigen-binding fragments thereof.

Another human anti-human CD27 antibody for use in the agonist is 2C2, which binds to a different epitope on CD27 than 1F5. The 2C2 antibody has VH and VL sequences shown in SEQ ID NOs: 3 and 4, respectively. Furthermore, another preferred antibody for use in the agonist is a recombinant anti-CD27 antibody comprising the heavy chain CDR1, CDR2 and CDR3 sequences and light chain CDR1, CDR2 and CDR3 sequences of 2C2, which are shown in SEQ ID NOs: 23, 24, 25 (heavy chain CDR1, CDR2 and CDR3 respectively) and SEQ ID NOs: 26, 27, 28 (heavy chain CDR1, CDR2 and CDR3 respectively). In a CD27 hyper-crosslinking agent as disclosed herein, the 2C2 antibody (having VH and VL sequences shown in SEQ ID NOs: 3 and 4, respectively) or an antibody comprising the 2C2 VH and VL CDRs (having sequences shown in SEQ ID NOs: 23-28), may be used as the second component, in combination with 1F5 antibody (or antibody comprising 1F5 CDRs) as the first component. Additionally or alternatively, the 2C2 antibody, or an antibody comprising the 2C2 CDRs, can be used as the first component in the CD27 agonist. Accordingly, in various embodiments, a CD27 agonist of the invention comprises a first component that is an anti-CD27 antibody or antigen-binding fragment thereof, comprising a VH and VL regions having amino acid sequences as shown in SEQ ID NOs: 1 and 2, respectively, or comprising VH and VL CDRs having amino acid sequences shown in SEQ ID NOs: 17-19 and 20-22, respectively. In one embodiment, the anti-CD27 antibody is an IgG1 antibody. Alternatively, the anti-CD27 antibody may be an F(ab)'2 fragment.

A CD27 agonist may comprise a second component that comprises one or more anti-CD27 antibodies or antigen-binding fragments thereof, comprising a VH and VL regions having amino acid sequences as shown in SEQ ID NOs: 3 and 4, respectively, or comprising VH and VL CDRs having amino acid sequences shown in SEQ ID NOs: 23-25 and 26-28, respectively.

A CD27 agonist may comprise a first component that is an anti-CD27 IgG1 antibody comprising a VH and VL regions having amino acid sequences as shown in SEQ ID NOs: 1 and 2, respectively (or comprising VH and VL CDRs having amino acid sequences shown in SEQ ID NOs: 17-19 and 20-22, respectively), and a second component that comprises an anti-CD27 sFv antibody fragment comprising VH and VL regions having amino acid sequences as shown in SEQ ID NOs: 1 and 2, respectively (or comprising VH and VL CDRs having amino acid sequences shown in SEQ ID NOs: 17-19 and 20-22, respectively).

A CD27 agonist may comprise a first component that is an anti-CD27 IgG1 antibody comprising a VH and VL regions having amino acid sequences as shown in SEQ ID NOs: 1 and 2, respectively (or comprising VH and VL CDRs having amino acid sequences shown in SEQ ID NOs: 17-19 and 20-22, respectively), and a second component that comprises an anti-CD27 sFv fragment comprising a VH and VL regions having amino acid sequences as shown in SEQ ID NOs: 3 and 4, respectively (or comprising VH and VL CDRs having amino acid sequences shown in SEQ ID NOs: 23-25 and 26-28, respectively).

A CD27 agonist may comprise a first component that is an anti-CD27 IgG1 antibody or F(ab)'2 fragment comprising a VH and VL regions having amino acid sequences as shown in SEQ ID NOs: 1 and 2, respectively (or comprising VH and VL CDRs having amino acid sequences shown in SEQ ID NOs: 17-19 and 20-22, respectively), and a second component that comprises one or more anti-CD27 Fab fragments comprising a VH and VL regions having amino acid sequences as shown in SEQ ID NOs: 1 and 2, respectively (or comprising VH and VL CDRs having amino acid sequences shown in SEQ ID NOs: 17-19 and 20-22, respectively). The second component may comprise three or more anti-CD27 Fab fragments comprising a VH and VL regions having amino acid sequences as shown in SEQ ID NOs: 1 and 2, respectively (or comprising VH and VL CDRs having amino acid sequences shown in SEQ ID NOs: 17-19 and 20-22, respectively).

Similar to the foregoing, the human anti-human CD27 antibodies 3H12, 2G9, 1H8, 3A10, 3H8 and IG5 (having VH and VL sequences as shown in SEQ ID NOs: 5-16) can be used as first or second components in CD27 agonists. Moreover, antibodies may comprise the heavy chain CDRs and light chain CDRs of any of 3H12, 2G9, 1H8, 3A10, 3H8 and 1G5. The heavy chain and light chain CDR1, CDR2 and CDR3 for these antibodies, which are underlined in the sequences shown in the Summary of Sequence Listing, can be determined by the ordinarily skilled artisan based on the VH and VL sequences and information known in the art for identifying CDRs. Moreover, the heavy and light chain CDR1, CDR2 and CDR3 for antibodies 3H12, 2G9, 1H8, 3A10, 3H8 and IG5 are shown as follows: 3H12 VH CDR1, CDR2, CDR3 in SEQ ID NOs: 17, 18 and 19, respectively; 3H12 VL CDR1, CDR2, CDR3 in SEQ ID NOs: 20, 21 and 22, respectively; 2G9 VH CDR1, CDR2, CDR3 in SEQ ID NOs: 37, 24 and 38, respectively; 2G9 VL CDR1, CDR2, CDR3 in SEQ ID NOs: 26, 27 and 28, respectively; 1H8 VH CDR1, CDR2, CDR3 in SEQ ID NOs: 39, 40 and 41, respectively; 1H8 VL CDR1, CDR2, CDR3 in SEQ ID NOs: 26, 27 and 42, respectively; 3A10 VH CDR1, CDR2, CDR3 in SEQ ID NOs: 43, 18 and 44, respectively; 3A10 VL CDR1, CDR2, CDR3 in SEQ ID NOs: 45, 27 and 46, respectively; 3H8 VH CDR1, CDR2, CDR3 in SEQ ID NOs: 47, 48 and 49, respectively; 3H8 VL CDR1, CDR2, CDR3 in SEQ ID NOs: 50, 51 and 52, respectively; IG5 VH CDR1, CDR2, CDR3 in SEQ ID NOs: 53, 54 and 55, respectively; 1G5 VL CDR1, CDR2, CDR3 in SEQ ID NOs: 56, 51 and 57, respectively.

A CD27 agonist that is a CD27 hyper-crosslinking agent may comprise a first component that is an anti-CD27 IgG1 antibody or F(ab)'2 fragment comprising a VH and VL regions having amino acid sequences as shown in SEQ ID NOs: 1 and 2, respectively (or comprising VH and VL CDRs having amino acid sequences shown in SEQ ID NOs: 17-19 and 20-22, respectively), and a second component that comprises a CD70 extracellular domain. Preferably, the CD70 extracellular domain has the amino acid sequence shown in SEQ ID NO: 31, corresponding to amino acid residues 27-176 of the mature human CD70 protein.

Various CD27 agonists are multi-specific agents. For example, a CD27 agonist that is a multi-specific agent may comprise a first component that is an anti-CD27 IgG1 antibody comprising VH and VL regions having amino acid sequences as shown in SEQ ID NOs: 1 and 2, respectively (or comprising VH and VL CDRs having amino acid sequences shown in SEQ ID NOs: 17-19 and 20-22, respectively), and a second component that comprises an anti-OX-40 sFv fragment. Non-limiting examples of anti-OX-40 antibodies that can be used in such a multi-specific agent include those disclosed in PCT Publications WO 2010/096418, WO 2012/027328, WO 2013/008171, WO 2013/038191 and WO 2013/068563.

A CD27 agonist of the invention that is a multi-specific agent preferably comprises a first component that binds to CD27 such as an anti-CD27 IgG1 antibody (for example comprising a VH and VL regions having amino acid sequences as shown in SEQ ID NOs: 1 and 2, respectively (or comprising VH and VL CDRs having amino acid sequences shown in SEQ ID NOs: 17-19 and 20-22, respectively)), and a second component that binds to PD-L1 such as an anti-PD-Ll sFv fragment. Non-limiting examples of anti-PD-Ll antibodies that can be used in such a multi-specific agent include those disclosed in PCT Publications WO 2007/005874, WO 2010/077634 and WO 2013/079174. Exemplary anti-PD-Ll VH and VL sequences that can be used to prepare an anti-PD-Ll sFv are shown in SEQ ID NOs: 32 and 33, respectively. Construction of a CD27 agonist comprising an anti-CD27 mAb linked to an anti-PD-L1 scFv is described further in Example 8 and the functional activity of this CD27 agonist is analyzed in Examples 9 and 10, thereby demonstrating the synergistic activity of the CD27 agonist as compared to co-administration of anti-CD27 antibodies with (unlinked) anti-PD-L1 antibodies.

Furthermore, a CD27 agonist that is a multi-specific agent may comprise a first component that binds to CD27 such as an anti-CD27 IgG1 antibody (for example comprising a VH and VL regions having amino acid sequences as shown in SEQ ID NOs: 1 and 2, respectively (or comprising VH and VL CDRs having amino acid sequences shown in SEQ ID NOs: 17-19 and 20-22, respectively)), and a second component that comprises the heavy and light chains (or heavy and light chain variable regions, or VH and VL CDRs thereof) of the anti-PD-1 antibody known in the art as Nivolumab, an anti-PD-1 antibody known in the art as Pembrolizumab, an anti-PD-L1 antibody known in the art as Durvelumab or an anti-CTLA-4 antibody known in the art as Ipilimumab. The full-length heavy chain and light chain amino acid sequences of Nivolumab are shown in SEQ ID NOs: 68 and 69, respectively. The full-length heavy chain and light chain amino acid sequences of Pembrolizumab are shown in SEQ ID NOs: 70 and 71, respectively. The full-length heavy chain and light chain amino acid sequences of Durvelumab are shown in SEQ ID NOs: 72 and 73, respectively. The full-length heavy chain and light chain amino acid sequences of Ipilimumab are shown in SEQ ID NOs: 74 and 75, respectively. The second component of the multi-specific agent can comprise, for example, the full length heavy and light chains of any of these antibodies, or the VH and VL sequences of these antibodies (e.g., in a scFv format) or the VH and VL CDRs of any of these antibodies. Moreover, use of sequences substantially homologous (e.g. at least 80%, 90%, 95%, 98% or 99% identical to the aforementioned sequences, e.g., SEQ ID NOs: 68-75 or the VH or VL regions thereof) are also encompassed.

A CD27 agonist that is a multi-specific agent may comprise a first component that binds to CD27 such as an anti-CD27 IgG1 antibody (for example comprising a VH and VL regions having amino acid sequences as shown in SEQ ID NOs: 1 and 2, respectively (or comprising VH and VL CDRs having amino acid sequences shown in SEQ ID NOs: 17-19 and 20-22, respectively)), and a second component that binds to an anti-Tumor Associated Antigen such as an anti-Tumor Associated Antigen sFv fragment. Non-limiting examples of anti-TAA antibodies that can be used in such a multi-specific agent include antibodies to EGFR, EGFRvIII, gp100 or Pmel17, HER2/neu, mesothelin, CEA, MARTI, MAGE-A1, MAGE-A2, MAGE-A3, MAGE-A4, MUC-1, GPNMB, HMW-MAA, TIM1, ROR, NY-ESO-1 and CD19. Exemplary anti-TIMl antibodies include those disclosed in PCT Publications WO 2004/084823. Exemplary anti-TIM1 VH and VL sequences that can be used to prepare an anti-TIM1 sFv are shown in SEQ ID NOs: 34 and 35, respectively.

A CD27 agonist that is a multi-specific agent may comprise a first component that binds to CD27 such as an anti-CD27 IgG1 antibody (for example comprising a VH and VL regions having amino acid sequences as shown in SEQ ID NOs: 1 and 2, respectively (or comprising VH and VL CDRs having amino acid sequences shown in SEQ ID NOs: 17-19 and 20-22, respectively)), and a second component that binds to OX-40 or a TAA such as an anti-OX-40 sFv fragment and/or an anti-TAA sFv fragment.

### V. Preparation of CD27 Agonists

The CD27 agonists can be prepared using one or more of a variety of methods well established in the art. For example, a CD27 agonist can be prepared by chemical conjugation, wherein the first component is linked (preferably covalently) to the second component through chemical conjugation. Alternatively, a CD27 agonist can be prepared by genetic engineering methods, wherein a nucleic acid encoding the first component is operatively linked to a nucleic acid encoding the second component such that a nucleic acid encoding a fusion protein comprising the first component linked to the second component is created. The nucleic acid encoding the fusion protein can be expressed recombinantly using standard recombinant DNA techniques and gene transfection methods as is well known in the art (e.g., Morrison, S. (1985) Science 229:1202).

For chemical conjugation, suitable reagents and methods are known in the art for coupling two or more moieties, in particular two or more antibodies, or fragments thereof, together. A variety of coupling or crosslinking agents are commercially available and can be used to conjugate the first and second component of the CD27 agonist. Non-limiting examples include Sulfo-SMCC, protein A, carboiimide, dimaleimide, dithio-bis-nitrobenzoic acid (DTNB), and N-succinimidyl-3-(2-pyridyldithio) propionate (SPDP). Sulfo-SMCC, SPDP and DTNB are preferred agents, with Sulfo-SMCC being particularly preferred. An exemplary coupling of F(ab)'2 antibody to Fab fragments using Sulfo-SMCC is described in detail in Reference Example 1. Other suitable procedures for crosslinking components (e.g., antibodies) with cross-linking agents are known in the art. See *e.g.,* Karpovsky, B. et al., (1984) J. Exp. Med. 160:1686; Liu, M. A. et al., (1985) Proc. Natl. Acad. Sci USA 82:8648; Segal, D. M. and Perez, P., U.S. Pat. No. 4,676,980; and Brennan, M. (1986) Biotechniques 4:424.

For genetic engineering, nucleic acid molecules encoding the light and heavy chain sequences of the anti-CD27 antibody, or fragment, that is the first component of the CD27 agonist can be inserted into an appropriate expression vector using standard recombinant DNA techniques. A nucleic acid molecule(s) encoding the second component of the CD27 agonist also can be inserted into the same expression vector, such that it is operatively linked (*e.g*., in-frame cloning) to the first component, thereby resulting in an expression vector that encodes a fusion protein that is the CD27 agonist. Preferably, the second component is operatively linked to the C-terminal region of the heavy chain of the anti-CD27 antibody that is the first component of the CD27 agonist. The genetic engineering of exemplary expression vectors encoding the VH and VL regions of an anti-CD27 antibody (first component) operatively linked to a sFv (second component) or CD27 ligand (second component) is described in detail in Example 2. Other suitable expression vectors and cloning strategies for preparing recombinant CD27 agonists in accordance with the invention are readily apparent to the ordinarily skilled artisan.

For expression of the CD27 agonists in host cells, the coding regions of the components of the agonist (e.g., heavy and light chain variable regions) are combined with cloned promoter, leader sequence, translation initiation, leader sequence, constant region, 3' untranslated, polyadenylation, and transcription termination, sequences to form expression vector constructs. These constructs can be used to express, for example, full length human IgG₁κ or IgG₄κ antibodies. Fully human, humanized and chimeric antibodies used in the agonists also include IgG2, IgG3, IgE, IgA, IgM, and IgD antibodies. Similar plasmids can be constructed for expression of other heavy chain isotypes, or for expression of antibodies comprising lambda light chains.

Following preparation of an expression vector encoding the CD27 agonist, the CD27 agonist can be expressed recombinantly in a host cell using standard transfection methods. For example, in one embodiment, nucleic acid encoding the CD27 agonist can be ligated into an expression vector such as a eukaryotic expression plasmid such as used by GS gene expression system disclosed in WO 87/04462, WO 89/01036 and EP 338 841 or other expression systems well known in the art. The purified plasmid with the cloned CD27 agonist gene can be introduced in eukaryotic host cells such as CHO-cells or NSO-cells or alternatively other eukaryotic cells like a plant derived cells, fungi or yeast cells. The method used to introduce these genes could be methods described in the art such as electroporation, lipofectin, lipofectamine or other. After introducing the expression vector in the host cells, cells expressing the CD27 agonist can be identified and selected. These cells represent the transfectomas that can then be amplified for their expression level and upscaled to produce CD27 agonists. Alternatively these cloned CD27 agonists can be expressed in other expression systems such as *E. coli* or in complete organisms or can be synthetically expressed. Recombinant CD27 agonists can be isolated and purified from these culture supernatants and/or cells.

A CD27 agonist, whether prepared by chemical conjugation or by genetic engineering, can be isolated and purified using one or more methodologies for protein purification well established in the art. Preferred methods for isolation and purification include gel filtration chromatography, affinity chromatography, anion-exchange chromatography and the like. A particularly preferred method is gel filtration chromatography, *e.g*., using a Superdex 200 column. Isolated and purified CD27 agonist can be evaluated using standard methods such as SDS-PAGE analysis.

### VI. Compositions

In another embodiment, the present invention provides a composition, *e.g*., a composition comprising one or a combination of CD27 agonists of the present invention, formulated together with a carrier (*e.g.,* a pharmaceutically acceptable carrier).

Pharmaceutical compositions of the invention also can be administered in combination therapy, *i.e*., combined with other agents. For example, the combination therapy can include a composition of the present invention with at least one or more additional therapeutic agents, such as anti-inflammatory agents, DMARDs (disease-modifying antirheumatic drugs), immunosuppressive agents, and chemotherapeutics. The pharmaceutical compositions of the invention can also be administered in conjunction with radiation therapy. Co-administration with other antibodies is also encompassed by the invention.

As used herein, the terms "carrier" and "pharmaceutically acceptable carrier" includes any and all solvents, salts, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (*e.g*., by injection or infusion). Depending on the route of administration, the active compound, *i.e.,* CD27 agonist, may be coated in a material to protect the compound from the action of acids and other natural conditions that may inactivate the compound.

Examples of adjuvants which may be used with the CD27 agonists of the present invention include: Freund's Incomplete Adjuvant and Complete Adjuvant (Difco Laboratories, Detroit, Mich.); Merck Adjuvant 65 (Merck and Company, Inc., Rahway, N.J.); AS-2 (SmithKline Beecham, Philadelphia, Pa.); aluminum salts such as aluminum hydroxide gel (alum) or aluminum phosphate; salts of calcium, iron or zinc; an insoluble suspension of acylated tyrosine; acylated sugars; cationically or anionically derivatised polysaccharides; polyphosphazenes; biodegradable microspheres; cytokines, such as GM-CSF, interleukin-2, - 7, -12, and other like factors; 3D-MPL; CpG oligonucleotide; and monophosphoryl lipid A, for example 3-de-O-acylated monophosphoryl lipid A.

MPL adjuvants are available from Corixa Corporation (Seattle, Wash; see, for example, U.S. Pat. Nos. 4,436,727; 4,877,611; 4,866,034 and 4,912,094). CpG-containing oligonucleotides (in which the CpG dinucleotide is unmethylated) are well known and are described, for example, in WO 96/02555, WO 99/33488 and U.S. Pat. Nos. 6,008,200 and 5,856,462. Immunostimulatory DNA sequences are also described, for example, by Sato et al., Science 273:352, 1996.

Further alternative adjuvants include, for example, saponins, such as Quil A, or derivatives thereof, including QS21 and QS7 (Aquila Biopharmaceuticals Inc., Framingham, Mass.); Escin; Digitonin; or Gypsophila or Chenopodium quinoa saponins; Montanide ISA 720 (Seppic, France); SAF (Chiron, California, United States); ISCOMS (CSL), MF-59 (Chiron); the SBAS series of adjuvants (e.g., SBAS-2 or SBAS-4, available from SmithKline Beecham, Rixensart, Belgium); Detox (Enhanzyn^{™}) (Corixa, Hamilton, Mont.); RC-529 (Corixa, Hamilton, Mont.) and other aminoalkyl glucosaminide 4-phosphates (AGPs); polyoxyethylene ether adjuvants such as those described in WO 99/52549A1; synthetic imidazoquinolines such as imiquimod [S-26308, R-837], (Harrison, et al., Vaccine 19: 1820-1826, 2001; and resiquimod [S-28463, R-848] (Vasilakos, et al., Cellular immunology 204: 64-74, 2000; Schiff bases of carbonyls and amines that are constitutively expressed on antigen presenting cell and T-cell surfaces, such as tucaresol (Rhodes, J. et al., Nature 377: 71-75, 1995); cytokine, chemokine and co-stimulatory molecules as either protein or peptide, including for example pro-inflammatory cytokines such as Interferon, GM-CSF, IL-1 alpha, IL-1 beta, TGF-alpha and TGF-beta, Th1 inducers such as interferon gamma, IL-2, IL-12, IL-15, IL-18 and IL-21, Th2 inducers such as IL-4, IL-5, IL-6, IL-10 and IL-13 and other chemokine and co-stimulatory genes such as MCP-1, MIP-1 alpha, MIP-1 beta, RANTES, TCA-3, CD80, CD86 and CD40L; immunostimulatory agents targeting ligands such as CTLA-4 and L-selectin, apoptosis stimulating proteins and peptides such as Fas; synthetic lipid based adjuvants, such as vaxfectin, (Reyes et al., Vaccine 19: 3778-3786, 2001) squalene, alpha-tocopherol, polysorbate 80, DOPC and cholesterol; endotoxin, [LPS], (Beutler, B., Current Opinion in Microbiology 3: 23-30, 2000); ligands that trigger Toll receptors to produce Th1-inducing cytokines, such as synthetic Mycobacterial lipoproteins, Mycobacterial protein p19, peptidoglycan, teichoic acid and lipid A; and CT (cholera toxin, subunits A and B) and LT (heat labile enterotoxin from E. coli, subunits A and B), heat shock protein family (HSPs), and LLO (listeriolysin O; WO 01/72329). These and various further Toll-like Receptor (TLR) agonists are described for example in Kanzler et al, Nature Medicine, May 2007, Vol 13, No 5. A preferred immunostimulatory agent for use in combination with a CD27 agonist of the invention is a TLR3 agonist, such as Poly IC.

A "pharmaceutically acceptable salt" refers to a salt that retains the desired biological activity of the parent compound and does not impart any undesired toxicological effects (see *e.g.,* Berge, S.M., et al. (1977) J. Pharm. Sci. 66:1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A composition of the present invention can be administered by a variety of methods known in the art. As will be appreciated by the skilled artisan, the route and/or mode of administration will vary depending upon the desired results. The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. *See, e.g.,* Sustained and Controlled Release Drug Delivery Systems, J.R. Robinson, ed., Marcel Dekker, Inc., New York, 1978.

To administer a compound of the invention by certain routes of administration, it may be necessary to coat the compound with, or co-administer the compound with, a material to prevent its inactivation. For example, the compound may be administered to a subject in an appropriate carrier, for example, liposomes, or a diluent. Acceptable diluents include saline and aqueous buffer solutions. Liposomes include water-in-oil-in-water CGF emulsions as well as conventional liposomes (Strejan et al. (1984) J. Neuroimmunol. 7:27).

Carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Dosage regimens are adjusted to provide the optimum desired response *(e.g.,* a therapeutic response). For example, a single bolus may be administered, several divided doses may be administered over time or the dose may be proportionally reduced or increased as indicated by the exigencies of the therapeutic situation. For example, the antibodies of the invention may be administered once or twice weekly by subcutaneous or intramuscular injection or once or twice monthly by subcutaneous or intramuscular injection.

It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

For the therapeutic compositions, formulations of the present invention include those suitable for oral, nasal, topical (including buccal and sublingual), rectal, vaginal and/or parenteral administration. The formulations may conveniently be presented in unit dosage form and may be prepared by any methods known in the art of pharmacy. The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, out of one hundred per cent, this amount will range from about 0.001 per cent to about ninety percent of active ingredient, preferably from about 0.005 per cent to about 70 per cent, most preferably from about 0.01 per cent to about 30 per cent.

Formulations of the present invention which are suitable for vaginal administration also include pessaries, tampons, creams, gels, pastes, foams or spray formulations containing such carriers as are known in the art to be appropriate. Dosage forms for the topical or transdermal administration of compositions of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents that delay absorption such as aluminum monostearate and gelatin.

When the compounds of the present invention are administered as pharmaceuticals, to humans and animals, they can be given alone or as a pharmaceutical composition containing, for example, 0.001 to 90% (more preferably, 0.005 to 70%, such as 0.01 to 30%) of active ingredient in combination with a pharmaceutically acceptable carrier.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient that is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts. A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved. In general, a suitable daily dose of a composition of the invention will be that amount of the compound that is the lowest dose effective to produce a therapeutic effect. Such an effective dose will generally depend upon the factors described above. It is preferred that administration be intravenous, intramuscular, intraperitoneal, or subcutaneous, preferably administered proximal to the site of the target. If desired, the effective daily dose of a therapeutic composition may be administered as two, three, four, five, six or more sub-doses administered separately at appropriate intervals throughout the day, optionally, in unit dosage forms. While it is possible for a compound of the present invention to be administered alone, it is preferable to administer the compound as a pharmaceutical formulation (composition).

Therapeutic compositions can be administered with medical devices known in the art. For example, in a preferred embodiment, a therapeutic composition of the invention can be administered with a needleless hypodermic injection device, such as the devices disclosed in U.S. Patent Nos. 5,399,163, 5,383,851, 5,312,335, 5,064,413, 4,941,880, 4,790,824, or 4,596,556. Examples of well-known implants and modules useful in the present invention include: U.S. Patent No. 4,487,603, which discloses an implantable micro-infusion pump for dispensing medication at a controlled rate; U.S. Patent No. 4,486,194, which discloses a therapeutic device for administering medicants through the skin; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments; and U.S. Patent No. 4,475,196, which discloses an osmotic drug delivery system. Many other such implants, delivery systems, and modules are known to those skilled in the art.

In certain embodiments, the antibodies of the invention can be formulated to ensure proper distribution *in vivo.* For example, the blood-brain barrier (BBB) excludes many highly hydrophilic compounds. To ensure that the therapeutic compounds of the invention cross the BBB (if desired), they can be formulated, for example, in liposomes. For methods of manufacturing liposomes, see, *e.g.,* U.S. Patents 4,522,811; 5,374,548; and 5,399,331. The liposomes may comprise one or more moieties that are selectively transported into specific cells or organs, thus enhance targeted drug delivery (*see, e.g.,* V.V. Ranade (1989) J. Clin. Pharmacol. 29:685). Exemplary targeting moieties include folate or biotin (see, *e.g.,* U.S. Patent 5,416,016 to Low et al.); mannosides (Umezawa et al., (1988) Biochem. Biophys. Res. Commun. 153:1038); antibodies (P.G. Bloeman et al. (1995) FEBSLett. 357:140; M. Owais et al. (1995) Antimicrob. Agents Chemother. 39:180); surfactant protein A receptor (Briscoe et al. (1995) Am. J. Physiol. 1233:134), different species of which may comprise the formulations of the inventions, as well as components of the invented molecules; p120 (Schreier et al. (1994) J. Biol. Chem. 269:9090); see also K. Keinanen; M.L. Laukkanen (1994) FEBS Lett. 346:123; J.J. Killion; I.J. Fidler (1994) Immunomethods 4:273. In one embodiment of the invention, the therapeutic compounds of the invention are formulated in liposomes; in a more preferred embodiment, the liposomes include a targeting moiety. In a most preferred embodiment, the therapeutic compounds in the liposomes are delivered by bolus injection to a site proximal to the tumor or infection. The composition must be fluid to the extent that easy syringability exists. It must be stable under the conditions of manufacture and storage and must be preserved against the contaminating action of microorganisms such as bacteria and fungi.

The ability of a compound to inhibit cancer can be evaluated in an animal model system predictive of efficacy in human tumors. Alternatively, this property of a composition can be evaluated by examining the ability of the compound to inhibit, such inhibition *in vitro* by assays known to the skilled practitioner. A therapeutically effective amount of a therapeutic compound can decrease tumor size, or otherwise ameliorate symptoms in a subject. One of ordinary skill in the art would be able to determine such amounts based on such factors as the subject's size, the severity of the subject's symptoms, and the particular composition or route of administration selected.

The composition must be sterile and fluid to the extent that the composition is deliverable by syringe. In addition to water, the carrier can be an isotonic buffered saline solution, ethanol, polyol (for example, glycerol, propylene glycol, and liquid polyetheylene glycol, and the like), and suitable mixtures thereof. Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition. Long-term absorption of the injectable compositions can be brought about by including in the composition an agent which delays absorption, for example, aluminum monostearate or gelatin.

When the active compound is suitably protected, as described above, the compound may be orally administered, for example, with an inert diluent or an assimilable edible carrier.

### VII. Uses and Methods of the Invention

The CD27 agonists of the invention are capable of stimulating T cell activity without the need for Fc receptor interaction. Accordingly, in one aspect, the invention provides an *in vitro* method of stimulating T cell activity, the method comprising contacting T cells with a CD27 agonist of the invention. Stimulation of T cell activity can be evaluated using any of a number of indicators of T cell activity known in the art. For example, enhancement of interferon-gamma production by OKT3-stimulated T cells can be used as a measure of T cell activation. A suitable assay is described further in Reference Example 4. Stimulation of T cell activity also can be evaluated using an NFkB-driven reporter gene system in a CD27-expressing cell, as described further in Example 5. Other suitable assays of T cell activation are well established in the art.

In another embodiment, the CD27 agonists of the invention can be used to treat and/or prevent (*e.g*., immunize against) a variety of diseases and conditions in which inducing or enhancing an immune response is desirable. Accordingly, the invention also provides a CD27 agonist of the invention for use in a method of inducing or enhancing an immune response against an antigen in a subject. The method may comprise administering to the subject a CD27 agonist of the invention in an amount effective to induce or enhance an immune response against an antigen in the subject. In one embodiment, the method further comprises administering the antigen to the subject. That is, the CD27 agonist and the antigen against which an immune response is desired can be co-administered to the subject. The CD27 agonist and antigen can be administered at the same time or alternatively, the CD27 agonist can be administered before or after the antigen is administered. Preferred antigens include tumor antigens and vaccine antigens (*e.g*., bacterial, viral or other pathogen antigens against which protective immunity is desired to be raised in a subject for purposes of vaccination).

One of the primary disease indications that can be treated using the CD27 agonists is cancer. In particular, a CD27 agonist that induces or enhances an immune response can be used in the treatment of cancer. Types of cancers include, but are not limited to, leukemia, acute lymphocytic leukemia, acute myelocytic leukemia, myeloblasts promyelocyte myelomonocytic monocytic erythroleukemia, chronic leukemia, chronic myelocytic (granulocytic) leukemia, chronic lymphocytic leukemia, mantle cell lymphoma, primary central nervous system lymphoma, Burkitt's lymphoma and marginal zone B cell lymphoma, Polycythemia vera Lymphoma, Hodgkin's disease, non-Hodgkin' s disease, multiple myeloma, Waldenstrom's macroglobulinemia, heavy chain disease, solid tumors, sarcomas, and carcinomas, fibrosarcoma, myxosarcoma, liposarcoma, chrondrosarcoma, osteogenic sarcoma, osteosarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon sarcoma, colorectal carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular tumor, lung carcinoma, small cell lung carcinoma, non small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma, retinoblastoma, nasopharyngeal carcinoma, esophageal carcinoma, basal cell carcinoma, biliary tract cancer, bladder cancer, bone cancer, brain and central nervous system (CNS) cancer, cervical cancer, choriocarcinoma, colorectal cancers, connective tissue cancer, cancer of the digestive system, endometrial cancer, esophageal cancer, eye cancer, head and neck cancer, gastric cancer, intraepithelial neoplasm, kidney cancer, larynx cancer, liver cancer, lung cancer (small cell, large cell), melanoma, neuroblastoma; oral cavity cancer(for example lip, tongue, mouth and pharynx), ovarian cancer, pancreatic cancer, retinoblastoma, rhabdomyosarcoma, rectal cancer; cancer of the respiratory system, sarcoma, skin cancer, stomach cancer, testicular cancer, thyroid cancer, uterine cancer, and cancer of the urinary system. Preferred cancers include CD27-expressing tumors selected from the group consisting of chronic lymphocytic leukemia, mantle cell lymphoma, primary central nervous system lymphoma, Burkitt's lymphoma and marginal zone B cell lymphoma.

Other disease indications for use of a CD27 agonist that induces or enhances an immune response include bacterial, fungal, viral and parasitic infectious diseases.

A CD27 agonist of the invention may be administered in combination with a vaccine, to enhance the immune response against the vaccine antigen, for example a tumor antigen (to thereby enhance the immune response against the tumor) or an antigen from an infectious disease pathogen (to thereby enhance the immune response against the infectious disease pathogen). A vaccine antigen can comprise, for example, an antigen or antigenic composition capable of eliciting an immune response against a tumor or against an infectious disease pathogen such as a virus, a bacteria, a parasite or a fungus. The antigen or antigens can be, for example, peptides/proteins, polysaccharides and/or lipids. The antigen or antigens be derived from tumors, such as the various tumor antigens previously disclosed herein. Alternatively, the antigen or antigens can be derived from pathogens such as viruses, bacteria, parasites and/or fungi, such as the various pathogen antigens previously disclosed herein. Additional examples of suitable pathogen antigens include, but are not limited to, the following:
Viral antigens or antigenic determinants can be derived from, for example,:
Cytomegalovirus ( especially Human, such as gB or derivatives thereof); Epstein Barr virus (such as gp350); flaviviruses (e.g. Yellow Fever Virus, Dengue Virus, Tick-borne encephalitis virus, Japanese Encephalitis Virus); hepatitis virus such as hepatitis B virus (for example Hepatitis B Surface antigen such as the PreS1, PreS2 and S antigens described in EP-A-414 374; EP-A-0304 578, and EP-A-198474), hepatitis A virus, hepatitis C virus and hepatitis E virus; HIV-1, (such as tat, nef, gp120 or gp160); human herpes viruses, such as gD or derivatives thereof or Immediate Early protein such as ICP27 from HSV1 or HSV2;
human papilloma viruses (for example HPV6, 11, 16, 18); Influenza virus (whole live or inactivated virus, split influenza virus, grown in eggs or MDCK cells, or Vero cells or whole flu virosomes (as described by Gluck, Vaccine, 1992,10, 915-920) or purified or recombinant proteins thereof, such as NP, NA, HA, or M proteins); measles virus; mumps virus;
parainfluenza virus; rabies virus; Respiratory Syncytial virus (such as F and G proteins);
rotavirus (including live attenuated viruses); smallpox virus; Varicella Zoster Virus (such as gpl, II and IE63); and the HPV viruses responsible for cervical cancer (for example the early proteins E6 or E7 in fusion with a protein D carrier to form Protein D-E6 or E7 fusions from HPV 16, or combinations thereof; or combinations of E6 or E7 with L2 (see for example WO 96/26277).

Bacterial antigens or antigenic determinants can be derived from, for example,: Bacillus spp., including B. anthracis (eg botulinum toxin); Bordetella spp, including B. pertussis (for example pertactin, pertussis toxin, filamenteous hemagglutinin, adenylate cyclase, fimbriae); Borrelia spp., including B. burgdorferi (eg OspA, OspC, DbpA, DbpB), B. garinii (eg OspA, OspC, DbpA, DbpB), B. afzelii (eg OspA, OspC, DbpA, DbpB), B. andersonii (eg OspA, OspC, DbpA, DbpB), B. hermsii; Campylobacter spp, including C. jejuni (for example toxins, adhesins and invasins) and C. coli; Chlamydia spp., including C. trachomatis (eg MOMP, heparin-binding proteins), C. pneumonie (eg MOMP, heparin-binding proteins), C. psittaci; Clostridium spp., including C. tetani (such as tetanus toxin), C. botulinum (for example botulinum toxin), C. difficile (eg clostridium toxins A or B); Corynebacterium spp., including C. diphtheriae (eg diphtheria toxin); Ehrlichia spp., including E. equi and the agent of the Human Granulocytic Ehrlichiosis; Rickettsia spp, including R.rickettsii; Enterococcus spp., including E. faecalis, E. faecium; Escherichia spp, including enterotoxic E. coli (for example colonization factors, heat-labile toxin or derivatives thereof, or heat-stable toxin), enterohemorragic E. coli, enteropathogenic E. coli (for example shiga toxin-like toxin); Haemophilus spp., including H. influenzae type B (eg PRP), non-typable H. influenzae, for example OMP26, high molecular weight adhesins, P5, P6, protein D and lipoprotein D, and fimbrin and fimbrin derived peptides (see for example US 5,843,464); Helicobacter spp, including H. pylori (for example urease, catalase, vacuolating toxin); Pseudomonas spp, including P. aeruginosa; Legionella spp, including L. pneumophila ; Leptospira spp., including L. interrogans; Listeria spp., including L. monocytogenes; Moraxella spp, including M catarrhalis, also known as Branhamella catarrhalis (for example high and low molecular weight adhesins and invasins); Morexella Catarrhalis (including outer membrane vesicles thereof, and OMP106 (see for example WO97/41731)); Mycobacterium spp., including M. tuberculosis (for example ESAT6, Antigen 85A, -B or -C), M. bovis, M. leprae, M. avium, M. paratuberculosis, M. smegmatis; Neisseria spp, including N. gonorrhea and N. meningitidis (for example capsular polysaccharides and conjugates thereof, transferrin-binding proteins, lactoferrin binding proteins, PilC, adhesins); Neisseria mengitidis B (including outer membrane vesicles thereof, and NspA ( see for example WO 96/29412); Salmonella spp, including S. typhi, S. paratyphi, S. choleraesuis, S. enteritidis; Shigella spp, including S. sonnei, S. dysenteriae, S. flexnerii; Staphylococcus spp., including S. aureus, S. epidermidis; Streptococcus spp, including S. pneumonie (eg capsular polysaccharides and conjugates thereof, PsaA, PspA, streptolysin, choline-binding proteins) and the protein antigen Pneumolysin (Biochem Biophys Acta, 1989,67,1007; Rubins et al., Microbial Pathogenesis, 25,337-342), and mutant detoxified derivatives thereof (see for example WO 90/06951; WO 99/03884); Treponema spp., including T. pallidum (eg the outer membrane proteins), T. denticola, T. hyodysenteriae; Vibrio spp, including V. cholera (for example cholera toxin); and Yersinia spp, including Y. enterocolitica (for example a Yop protein), Y. pestis, Y. pseudotuberculosis.

Parasitic/fungal antigens or antigenic determinants can be derived from, for example,: Babesia spp., including B. microti; Candida spp., including C. albicans; Cryptococcus spp., including C. neoformans; Entamoeba spp., including E. histolytica; Giardia spp., including ;G. lamblia; Leshmania spp., including L. major; Plasmodium. faciparum (MSP1, AMA1, MSP3, EBA, GLURP, RAP1, RAP2, Sequestrin, PfEMP1, Pf332, LSA1, LSA3, STARP, SALSA, PfEXPl, Pfs25, Pfs28, PFS27/25, Pfsl6, Pfs48/45, Pfs230 and their analogues in Plasmodium spp.); Pneumocystis spp., including P. ;carinii; Schisostoma spp., including S. mansoni; Trichomonas spp., including T. vaginalis; Toxoplasma spp., including T. gondii (for example SAG2, SAG3, Tg34); Trypanosoma spp., including T. cruzi.

It will be appreciated that in accordance with this aspect of the present invention antigens and antigenic determinants can be used in many different forms. For example, antigens or antigenic determinants can be present as isolated proteins or peptides (for example in so-called "subunit vaccines") or, for example, as cell-associated or virus-associated antigens or antigenic determinants (for example in either live or killed pathogen strains). Live pathogens will preferably be attenuated in known manner. Alternatively, antigens or antigenic determinants may be generated *in situ* in the subject by use of a polynucleotide coding for an antigen or antigenic determinant (as in so-called "DNA vaccination"), although it will be appreciated that the polynucleotides which can be used with this approach are not limited to DNA, and may also include RNA and modified polynucleotides as discussed above.

A vaccine antigen can also be targeted, for example to particular cell types or to particular tissues. For example, the vaccine antigen can be targeted to Antigen Presenting Cells (APCs), for example by use of agents such as antibodies targeted to APC-surface receptors such as DEC-205, for example as discussed in WO 2009/061996 (Celldex Therapeutics, Inc), or the Mannose Receptor (CD206) for example as discussed in WO 03040169 (Medarex, Inc.).

For use in therapy, the CD27 agonists of the invention can be administered to a subject directly (*i.e., in vivo*)*,* either alone or with other therapies such as an immunostimulatory agent, a vaccine, chemotherapy or radiation therapy. In all cases, the CD27 agonists and other therapies are administered in an effective amount to exert their desired therapeutic effect. The term "effective amount" refers to that amount necessary or sufficient to realize a desired biologic effect. For example, an effective amount could be that amount necessary to eliminate a tumor, cancer, or bacterial, viral or fungal infection. The effective amount for any particular application can vary depending on such factors as the disease or condition being treated, the particular antibody being administered, the size of the subject, or the severity of the disease or condition. One of ordinary skill in the art can empirically determine the effective amount of a particular molecule without necessitating undue experimentation.

Preferred routes of administration for vaccines include, for example, injection (e.g., subcutaneous, intravenous, parenteral, intraperitoneal, intrathecal). The injection can be in a bolus or a continuous infusion. Other routes of administration include oral administration.

CD27 agonists of the invention also can be coadministered with adjuvants and other therapeutic agents. It will be appreciated that the term "coadministered" as used herein includes any or all of simultaneous, separate, or sequential administration of the antibodies and conjugates of the present invention with adjuvants and other agents, including administration as part of a dosing regimen. The CD27 agonists are typically formulated in a carrier alone or in combination with such agents. Examples of such carriers include solutions, solvents, dispersion media, delay agents, emulsions and the like. The use of such media for pharmaceutically active substances is well known in the art. Any other conventional carrier suitable for use with the molecules falls within the scope of the instant invention.

Suitable agents for co-administration with the CD27 agonists include other antibodies, cytotoxins and/or drugs, as well as adjuvants, immunostimulatory agents and/or immunosuppressive agents. In one embodiment, the agent is a chemotherapeutic agent. The CD27 agonists can be administered in combination with radiation.

Chemotherapeutic agents suitable for coadministration with the CD27 agonists of the invention in the treatment of tumors include, for example: taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Further agents include, for example, antimetabolites (*e.g*., methotrexate, 6-mercaptopurine, 6-thioguanine, cytarabine, 5-fluorouracil decarbazine), alkylating agents (*e.g*., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g*., daunorubicin (formerly daunomycin) and doxorubicin), antibiotics (*e.g*., dactinomycin (formerly actinomycin), bleomycin, mithramycin, and anthramycin (AMC)), and anti-mitotic agents (*e.g*., vincristine and vinblastine) and temozolomide.

Agents that delete or inhibit immunosuppressive activities, for example, by immune cells (for example regulatory T-cells, NKT cells, macrophages, myeloid-derived suppressor cells, immature or suppressive dendritic cells) or suppressive factors produced by the tumor or host cells in the local microenvironment of the tumor (for example, TGFβ, indoleamine 2,3 dioxygenase - IDO), may also be administered with the CD27 agonists of the invention. Such agents include antibodies and small molecule drugs such as IDO inhibitors such as 1 methyl tryptophan or derivatives.

Suitable agents for coadministration with CD27 agonists of the invention for treatment of such immune disorders include for example, immunosuppressive agents such as rapamycin, cyclosporin and FK506; anti-TNFα agents such as etanercept, adalimumab and infliximab; and steroids. Examples of specific natural and synthetic steroids include, for example: aldosterone, beclomethasone, betamethasone, budesonide, cloprednol, cortisone, cortivazol, deoxycortone, desonide, desoximetasone, dexamethasone, difluorocortolone, fluclorolone, flumethasone, flunisolide, fluocinolone, fluocinonide, fluocortin butyl, fluorocortisone, fluorocortolone, fluorometholone, flurandrenolone, fluticasone, halcinonide, hydrocortisone, icomethasone, meprednisone, methylprednisolone, paramethasone, prednisolone, prednisone, tixocortol and triamcinolone.

Suitable agents for coadministration with the CD27 agonists of the invention for inducement or enhancement of an immune response include, for example, adjuvants and/or immunostimulatory agents, non-limiting examples of which have been disclosed hereinbefore. A preferred immunostimulatory agent is a TLR3 agonist, such as Poly IC.

The present invention is further illustrated by the following examples, which should not be construed as further limiting.

### EXAMPLES

### Reference Example 1: Construction of CD27 Agonists by Chemical Conjugation

In this example, an exemplary CD27 agonist was prepared by chemical conjugation. A F(ab)'2 form of the human anti-human CD27 monoclonal antibody 1F5 (described further in U.S. Patent Publications 20110274685 and 20120213771), having V_{H} and V_{L} sequences shown in SEQ ID NOs: 1 and 2, respectively, was used as the first component in the CD27 agonist. The second component of the CD27 agonist was a Fab fragment of the same antibody, 1F5, thus also having V_{H} and V_{L} sequences shown in SEQ ID NOs: 1 and 2, respectively, but lacking the ability to bind to Fc receptors.

1F5 Fab fragments were linked to F(ab)'2 fragments of 1F5 IgG antibody by chemical conjugation as follows: A 20 fold molar excess of Sulfo-SMCC was added to 2 mgs of 1F5 F (ab)'2, and incubated at room temperature for 3 hours. Excess unreacted Sulfo-SMCC was removed using a size exclusion column. Meanwhile, 2 mgs of 1F5 F(ab)'2 was reduced with 10 mM 2-Mercaptoethyl amine HCL (2-MEA) for 1 hour at 37 °C. The reaction mixture was loaded onto a size exclusion column, equilibrated with 0.1 M phosphate, 2.5mM EDTA pH 7.4 buffer. The Fab-SH was collected on ice. The maleimide activated 1F5 F (ab)'2 was added to the Fab-SH and the reaction mixture was incubated overnight at 2-8 °C. The reaction mixture was concentrated and loaded onto a sanitized Superdex 200 column equilibrated with phosphate buffered saline. The fractions collected and were analyzed on SDS-PAGE.

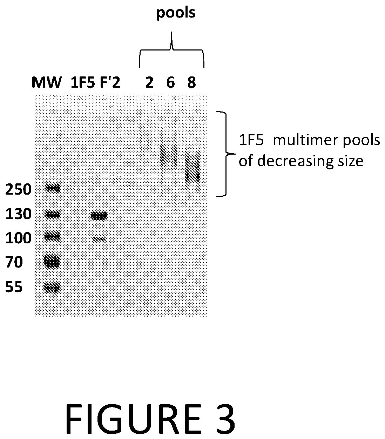

To analyze the resultant conjugates, standard SDS-PAGE was carried out under non-reducing conditions. The results are shown in Figure 3, which demonstrates that 1F5 multimer pools of decreasing size are detectable, thereby demonstrating that the chemical conjugation was successful. Size estimates for the 1F5 multimers (herein denoted "1F5x1F5") based on the SDS-PAGE analysis are as follows:

| Fraction | Size |
|---|---|
| # 2 | > 250Kd (Fab6, >Fab6) |
| # 6 | > 250Kd(Fab 5, >Fab5) |
| # 8 | > 250Kd (Fab4, >Fab4) |
| # 10 | 250 and >250Kd (Fab3, Fab4) |
| # 12 | 130, 250 and >250Kd (Fab2, Fab3, Fab4) |
| # 16 | 130 and 250Kd (Fab2 and Fab3) |

### Example 2: Construction of CD27 Agonists by Genetic Engineering

In this example, exemplary CD27 agonists were prepared by genetic engineering using standard recombinant DNA techniques. A full-length IgG form of the human anti-human CD27 monoclonal antibody 1F5 (described further in U.S. Patent Publications 20110274685 and 20120213771), having V_{H} and V_{L} sequences shown in SEQ ID NOs: 1 and 2, respectively, was used as the first component in the CD27 agonist. Nucleic acids encoding the heavy and light chains of full-length 1F5 were each cloned separately into a single expression vector, with each chain under the control of separate CMV promoters. The expression vector also encoded glutamine synthetase (GS) for selection purposes.

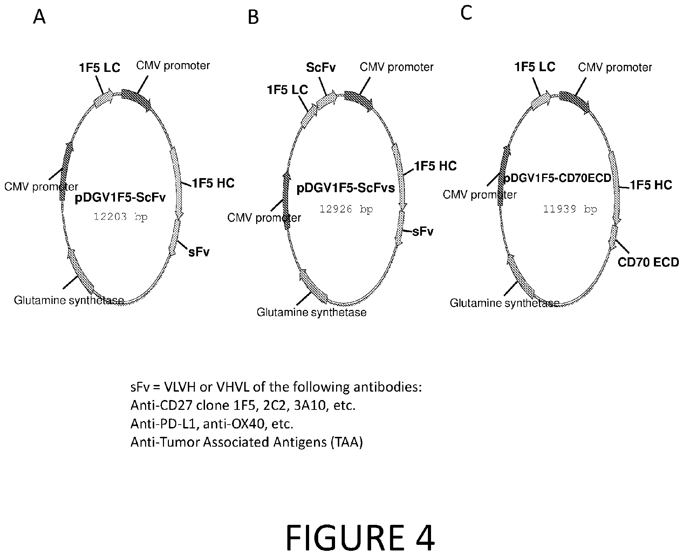

In a first set of vectors, the second component of the CD27 agonist was a single chain Fv (sFv) antibody construct. This type of expression vector, referred to as pDGV1F5-sFv or pDGV1F5-sFvs, is illustrated schematically in Figures 4A and 4B. The sFv can be, for example, a 1F5 sFv, having the same V_{H} and V_{L} as the full-length 1F5 used as the first component. Alternatively, the sFv can be from a different anti-CD27 antibody than 1F5, such as the human anti-human antibody 2C2 (described further in U.S. Patent Publications 20110274685 and 20120213771), having V_{H} and V_{L} sequences shown in SEQ ID NOs: 3 and 4, respectively. Still further, the sFv can be from an antibody that binds an antigen other than CD27 on T cells (*e.g.,* OX-40), immune cells (*e.g.,* PDL1) or tumor cells (*e.g.,* TAA). Exemplary amino acid sequences for anti-PD-L1 VH and VL chains, which can be used to prepare a sFv, are shown in SEQ ID NOs: 32 and 33, respectively. Exemplary amino acid sequences for anti-TIMl (a Tumor Associated Antigen) VH and VL chains, which can be used to prepare a sFv, are shown in SEQ ID NOs: 34 and 35, respectively. In the expression vector, a nucleic acid encoding the sFv was cloned into the vector such that the sFv was operatively linked to the C-terminal end of the full-length heavy chain of 1F5.

In another type of vector, the second component of the CD27 agonist was a CD27 ligand, in particular the extracellular domain of CD70 (CD70ECD). For the CD70ECD, amino acids 27-176 of the mature CD70 protein, having the amino acid sequence shown in SEQ ID NO: 31, were used. This type of expression vector, referred to as pDGV1F5-CD70ECD, is illustrated schematically in Figure 4C. In the expression vector, a nucleic acid encoding the extracellular domain of CD70 was cloned into the vector such that the CD70ECD was operatively linked to the C-terminal end of the full-length heavy chain of 1F5. The genetic fusion proteins prepared are summarized in Table 1 as follows:

**Table 1 - Summary of genetically derived CD27 agonists.**

| Name | CD27 Agonist Type | Description of the fusion protein |
|---|---|---|
| 1F5mAb-1F5sFv | CD27 crosslinking agent | Two 1F5 single-chain Fv fragments (sFvs) in VL-VH orientation, each genetically fused to the C-terminus of one of the two heavy chains of mAb 1F5 (IgG1κ) such that one sFv is fused to each heavy chain |
| 1F5mAb-2C2sFv | CD27 crosslinking agent | Two 2C2 single-chain Fv fragments (sFvs) in VL-VH orientation, each genetically fused to the C-terminus of one of the two heavy chains of mAb 1F5 (IgG1κ) such that one sFv is fused to each heavy chain |
| 1FSmAb-2C2sFv-3A10sFv | CD27 crosslinking agent | Two 2C2 single-chain Fv fragments (sFvs) in VL-VH orientation, each genetically fused to the C-terminus of one of the two heavy chains of mAb 1F5 (IgG1κ), and two 3A10 single-chain Fv fragments (sFvs) in VL-VH orientation, each genetically fused to the C-terminus of one of the two light chains of mAb 1F5 (IgG1κ) such that one sFv is fused to each heavy or light chain |
| 1F5mAb-CD70 | CD27 crosslinking agent | Two CD70 extra-cellular domain (ECD) fragments, each genetically fused to the C-terminus of one of the two heavy chains of mAb 1F5 (IgG1κ) such that one CD70 ECD fragment is fused to each heavy chain |
| 1F5mAb-αPDL1sFv | Multi -specific agent | Two anti-PDL1 single-chain Fv fragments (sFvs), specific for both mouse and human PDL1, in VL-VH orientation, each genetically fused to the C-terminus of one of the two heavy chains of mAb 1F5 (IgG1κ) such that one sFv is fused to each heavy chain |

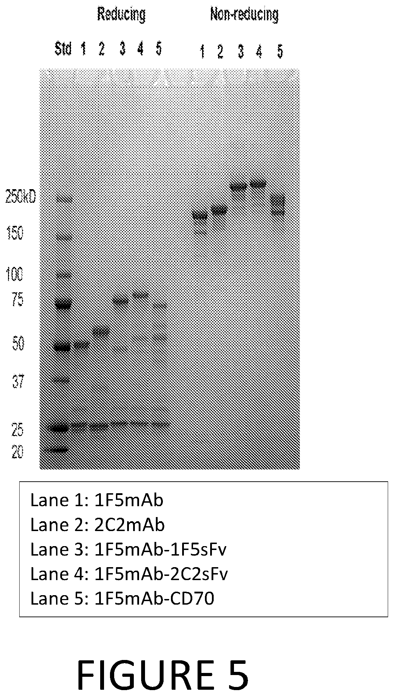

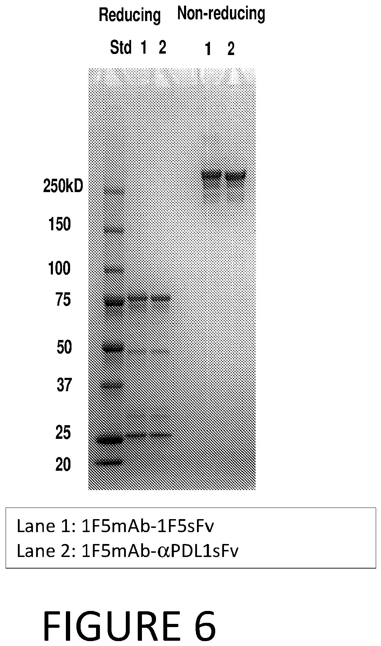

To analyze the resultant genetic fusion proteins (conjugates), standard SDS-PAGE (4-15% Tris-HCl; 2 µg protein/lane) was carried out under non-reducing and reducing conditions. The results are shown in Figure 5 and Figure 6. In particular, Figure 5 lanes 3, 4 and 5 of the non-reducing gel show the presence of the conjugated 1F5mAb-1F5sFv, 1F5mAb-2C2sFv and 1F5mAb-CD70 agonists, respectively. Figure 6 lanes 1 and 2 of the non-reducing gel show the presence of the conjugated 1F5mAb-1F5sFv and 1F5mAb-αPDL1sFv, respectively. The results shown in Figures 5 and 6 thereby demonstrate that the genetic engineering and recombinant expression of the CD27 agonist genetic fusion proteins (conjugates) was successful.

### Example 3: Binding Activity of CD27 Agonists

In this example, the binding activity of the CD27 agonists prepared as described in Example 2 was examined. Binding to recombinant CD27 (rCD27) was tested by standard ELISA. Human CD27 was captured on ELISA plates coated with anti-FLAG antibody, followed by incubation with CD27 agonists. A goat anti-human IgG Fc-HRP (horseradish peroxidase) antibody and substrate Super Blue TMB were used for detection.

The CD27 agonists tested were 1F5mAb-1F5sFv (sFv), 1F5mAb-2C2sFv (sFv) and 1F5mAb-CD70. The full-length, unconjugated IgG form of the anti-human CD27 monoclonal antibodies 1F5 and 2C2 were used as positive controls. Binding to a mutant form of soluble CD27, in which the arginine at position 87 was substituted with alanine (R87A), which is within the 1F5 binding site, was also tested.

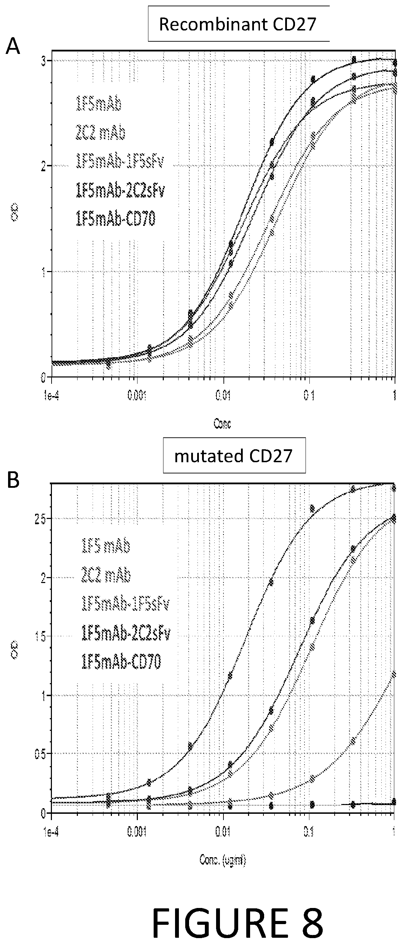

The results are shown in Figures 8A (rCD27) and 8B (rCD27 with a mutation introduced that eliminates 1F5 binding but does not affect 2C2 binding to CD27). The results in Figure 8A demonstrate that all the constructs tested bound well to CD27 captured on the microtiter plates. Furthermore, the results in Figure 8B demonstrate that neither 1F5 nor 1F5mAb-1F5sFv binds to the CD27 mutant, but that 2C2, 1F5mAb-2C2sFv and IF5mAb-CD70 still do.

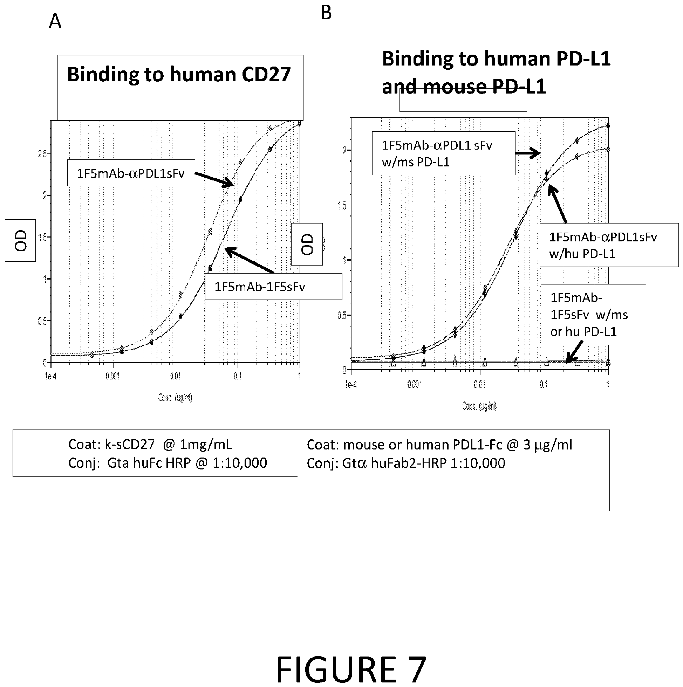

In another series of experiments, the 1F5mAb-1F5sFv sFv conjugate and the 1F5mAb-αPDL1sFv sFv conjugate were tested in binding assays against human CD27 and against human and mouse PD-L1, as the anti-PD-Ll sFv is known to bind to both human and murine PD-L1. The results are shown in Figures 7A (CD27) and 7B (PD-L1). The results shown in Figure 7A demonstrate that both the 1F5mAb-1F5sFv conjugate and the 1F5mAb-αPDL1sFv conjugate bound well to human CD27 captured on the microtiter plate. The results shown in Figure 7B demonstrate that the 1F5mAb-αPDL1sFv conjugate bound well to both mouse and human PD-L1, whereas the 1F5mAb-1F5sFv conjugate did not bind to either mouse or human PD-L1 (as expected since it does not contain any PD-L1 binding moieties).

The experiments described herein demonstrate that the CD27 agonists exhibit binding to CD27. Furthermore, the multi-specific CD27 agonist that contains both an anti-CD27 moiety and an anti-PD-Ll moiety exhibits binding to both CD27 and to PD-L1.

### Reference Example 4: Ability of CD27 Agonists to Costimulate Purified T cells

In this example, the T cell activation ability of the CD27 agonists prepared as described in Reference Example 1 was examined using cytokine (interferon-gamma (IFN-γ) or IL-2) release assays. Production of cytokine by purified human T cells stimulated with microtiter plate-bound OKT3, with or without soluble fractions of 1F5 multimers (CD27 agonists), was used as the indicator of T cell activation. T cells were isolated from PBMCs. The T cells (100,000 cells/well) were incubated with dry coated OKT3 and dilutions of the soluble fractions of multimers. After 72 hours, cell culture supernatants were harvested and analyzed using R&D Systems Human IFN- γ or IL-2 DuoSet ELISA kit. The positive control was T cells incubated with dry coated OKT3 and full-length unconjugated anti-CD27 antibody 1F5 also dry-coated on the microtiter plate. The negative controls were T cells incubated with either dry coated OKT3 and dry-coated human IgG (hIgG), or with dry-coated OKT3 alone.

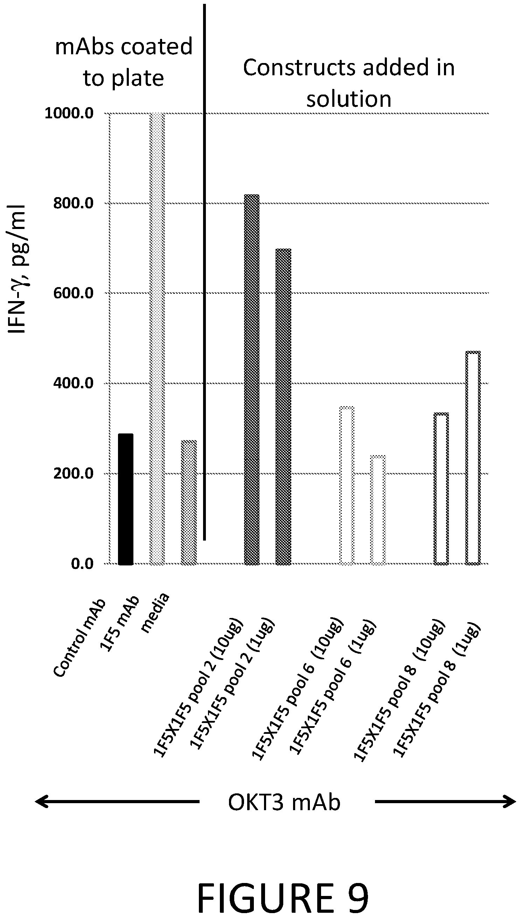

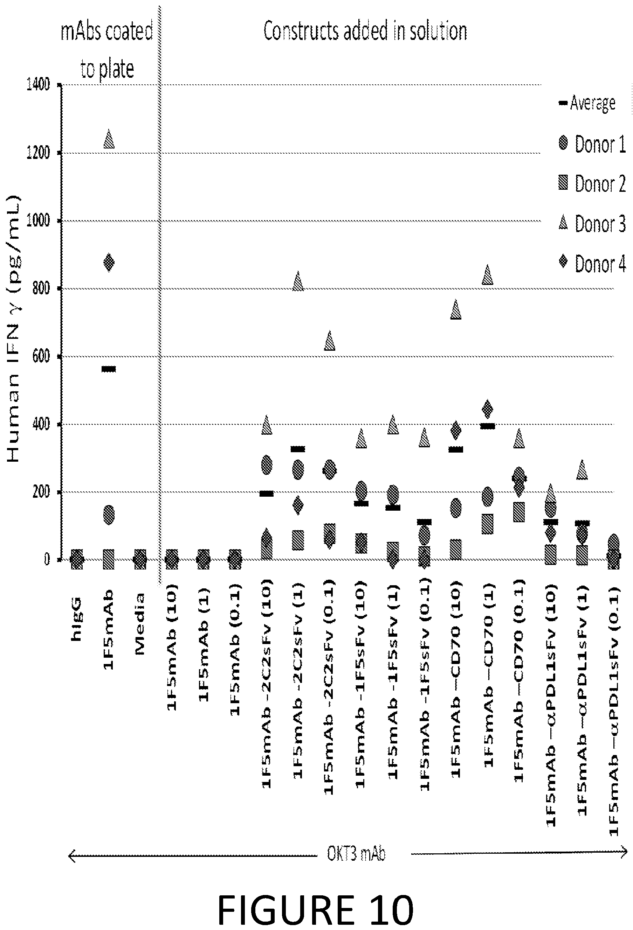

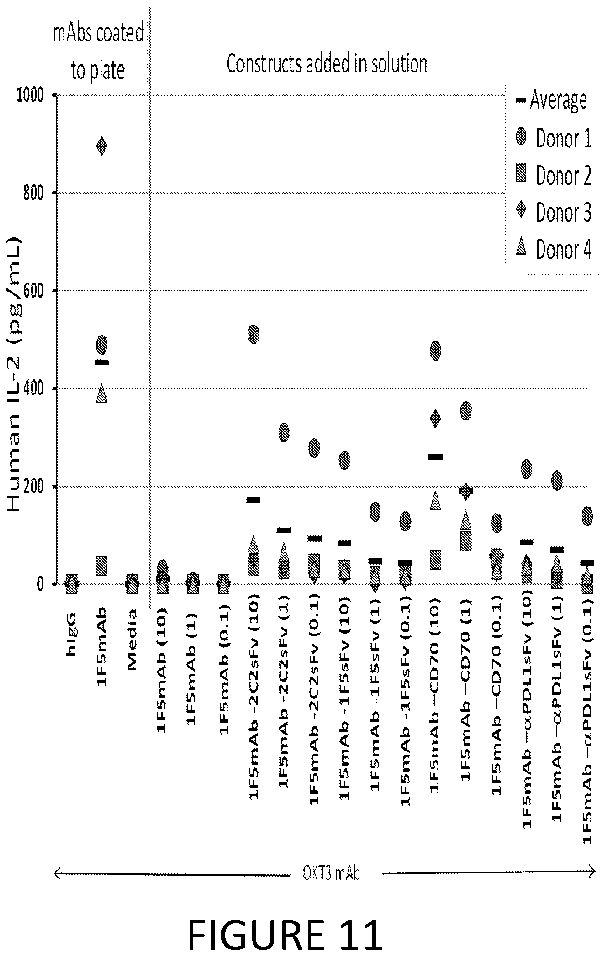

The results are shown in Figures 9-11, which demonstrate that the soluble fractions of various 1F5 multimers (CD27 agonists) were capable of stimulating T cell activation, as measured by enhanced IFN- γ or IL-2 production. Figure 9 demonstrates the activity of the chemically conjugated CD27 agonists (IF5×1F5), and these data imply that higher order conjugates, in particular those in fraction #2, had the greatest agonist activity. Figures 10 and 11 demonstrate the activity of the genetically engineered CD27 agonists as measured by enhanced IFN-γ or IL-2 production, respectively. For these data, the control OKT3 + human IgG was subtracted to clearly show the enhanced activity of the CD27 agonists using T cells from 4 separate donors. The letter "S" denotes the construct was added in solution. The values in parentheses are the final concentration of the construct in micrograms/ml.

### Example 5: Ability of CD27 Agonists to Stimulate NFkB Signaling Pathways

The functional activity of the CD27 agonists was also assessed using a reporter cell line system. A stable cell line was created for studying NFkB signaling pathways activated by CD27 binding agents. A commercially available cell line was purchased from Signosis, Inc. (Santa Clara, CA). Signosis established the NFkB luciferase reporter stable cell line through stable transfection of human embryonic kidney 293 cells (HEK-293) with pTA-NFkB -luciferase reporter vector, which contains 4 repeats of NFkB binding sites, a minimal promoter upstream of the firefly luciferase coding region. Therefore, the cell line can be used as a reporter system for monitoring the activation of NFkB triggered by stimuli treatment. The HEK-293 cells do not naturally express CD27, therefore, the cell line was stably transfected with human CD27 to create the reported cells used for analyzing CD27-mediated NFkB signaling.

To perform the hCD27/NFkB/293/Luciferase cell line assay, 1×10⁴ cells (in 100 µl complete media) were seeded in each well of a 96 well, flat-bottomed polystyrene plate. The cells were incubated overnight at 37 °C (5% CO₂) (100 µl/well final volume). The following day, dilutions of CD27 agonists were prepared and 10 µl were dispensed per well (10 µg/ml final concentration). The cells were incubated with the CD27 agonist for 6 hours at 37 °C (5% CO₂). After the 6 hour incubation, the media was removed and 100 µl 1xDPBS was dispensed as a wash step and then removed. Next, 20 µl of 1x Passive lysis buffer was dispensed and the cells incubated at room temperature for 15 minutes. Next, 100 µl luciferase substrate (reconstituted with luciferase buffer) was dispensed and pipetted gently up and down to mix. Finally, 100 µl of supernatant was transferred to an OptiPlate and read immediately on the "Victor" Luminometer.

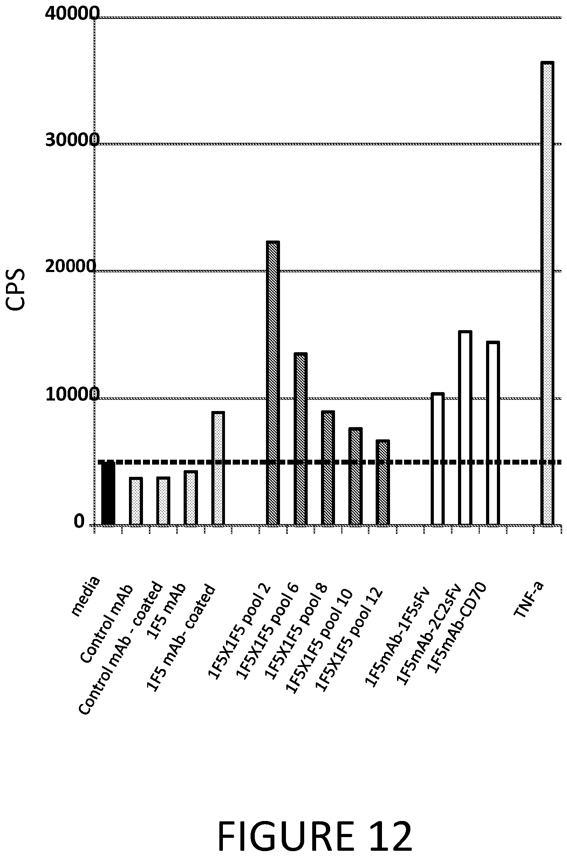

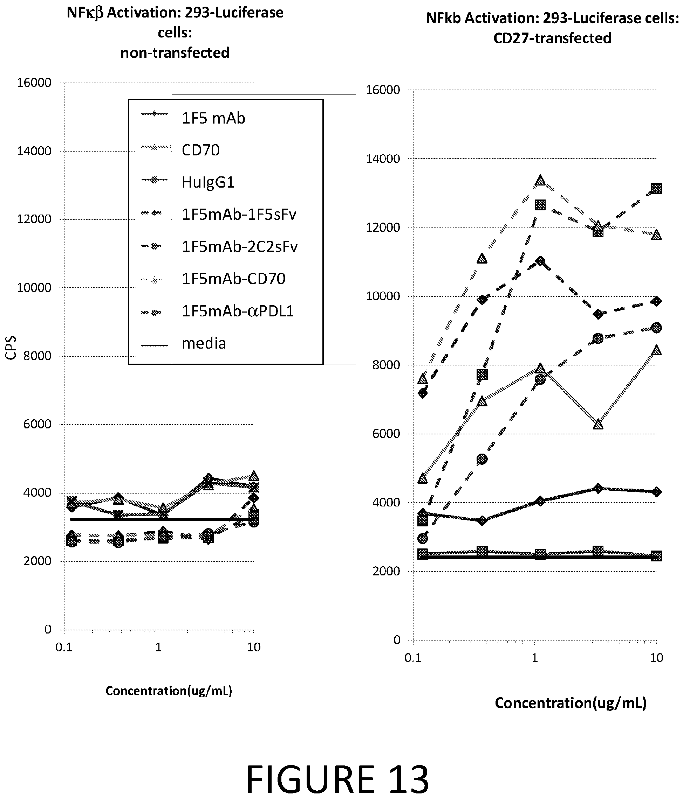

The effects of the CD27 agonists on reporter gene expression are shown in Figures 12 and 13. The reporter cell line was not stimulated above the baseline NFκB activity with the 1F5 mAb. However, the chemically-conjugated 1F5 multimers (1F5x1F5), and the genetically derived fusion protein agonists show significant NFκB activation. Interestingly, for the chemical conjugate, the level of activation was directly associated with increasing level of multimerization. The data also show that CD27 agonists composed of more than one specificity for CD27 (1F5 and 2C2 or 1F5 and CD70) were more potent than agents with a single specificity (1F5 x 1F5) using the NFkB reporter cell assay. The 1F5mAb-αPDL1sFv was also able to effectively cross-link CD27 because these reporter cells express PD-L1 and thus demonstrates that CD27 signaling can occur when the CD27 agonist binds with a non-CD27 molecule as well as when it binds to a CD27 molecule.

### Example 6: Ability of CD27 Agonists to Stimulate T cell Activation In Vivo

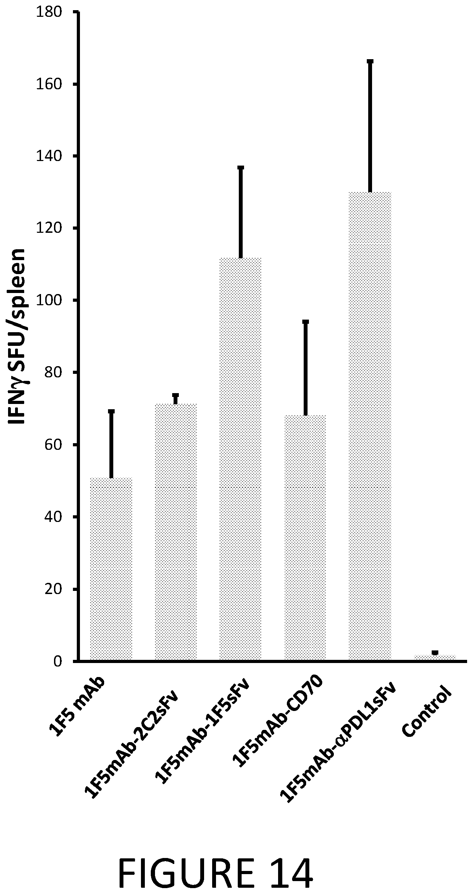

The functional activity of the CD27 agonists was also assessed using an *in vivo* model of human CD27-expressing transgenic mice. For these studies, groups of human CD27 transgenic mice were administered 5 milligrams of chicken ovalbumin and 100 micrograms of various CD27 agonists or controls by intraperitoneal injection. After 7 days, the spleen cells were isolated and the T cells were stimulated *in vitro* with SIINFEKL peptide (SEQ ID NO: 36). The activation status of T cells was measured using IFN-γ ELISPOT assays. Figure 14 shows that T cells from mice given the mutimeric constructs had higher antigen-specific IFN-γ production than T cells from mice treated with the CD27 antibody, 1F5 or controls.

### Example 7: Ability of CD27 Agonists to Mediate Anti-Tumor Activity

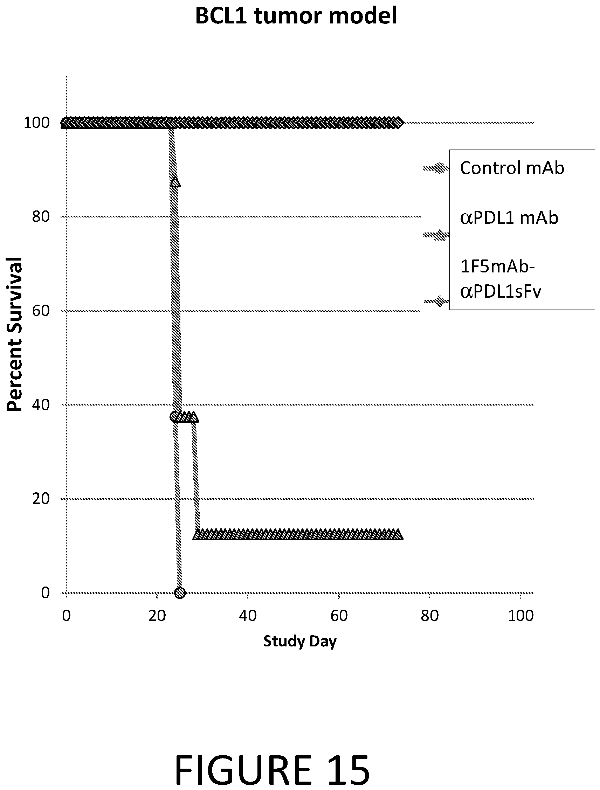

To test the anti-tumor activity of the CD27 multimeric agonists, the BCL1 lymphoma model was used in human CD27 transgenic mice. For these studies, 10 million BCL1 tumor cells are transferred by intravenous administration into human CD27 transgenic mice (Balb/C strain). Mice were treated by intraperitoneal injection of 100 micrograms of control antibody, anti-PD-Ll antibody or 1F5mAb-αPDL1sFv on days 3, 5, 7, 9, 11 and then followed for survival. Figure 15 shows that mice treated with the 1F5mAb-αPDL1sFv were protected from the BCL1 tumor challenge as compared to controls.

### Example 8: Construction and Expression of Anti-CD27 x Anti-PD-L1 Agonist

This example describes in further detail the construction and expression of the multispecific agent comprising the 1F5 anti-CD27 mAb genetically linked to an anti-PD-Ll sFv (as described above in Example 2), referred to herein 1F5mAb-αPDL1sFv.

The anti-PD-Ll VL and VH nucleotide sequence (encoding the amino acid sequences shown in SEQ ID NOs: 33 and 32, respectively) was synthesized with a [GGGGS]x3 linker in between the two variable regions, a [GGGGS]x1 linker at the N-terminus and flanking cloning sites for restriction enzymes Xhol and EcoRI. This 775 bp anti-PD-Ll ScFv fragment was then linked to the C-terminal end of the heavy chain of the 1F5 anti-CD27 monoclonal antibody. The 1F5 heavy chain sequence had previously been cloned into the pDGV vector (Lonza) by a three fragment ligation. This vector also carried the 1F5 light chain sequence. Following insertion of the anti-PD-Ll scFv sequence at the C-terminal end of the 1F5 heavy chain, the resultant expression plasmid (pDGV1F5-anti-PD-L1) was validated by restriction enzyme digestion and sequencing of the 1F5 HC - antiPD-L1 VL junction region. The structure of this plasmid is shown schematically in Figure 4A.

To generate the 1F5 x anti-PD-Ll bispecific antibody, 293 Freestyle cells (Invitrogen) were transfected with pDGV1F5-anti-PD-L1 plasmid using the polyethyleneimine (PEI) plus valproic acid (VPA) method known in the art. The bispecific antibody was then purified from the transfected cell culture medium using a MabSelect Sure (GE Life Sciences) protein A column. The bispecific antibody was analyzed in a 4-15% Tris-HCl SDS-PAGE gel, which showed the correct molecular weight (Figure 6). The bispecific antibody also was analyzed by ELISA for the binding to soluble CD27 and PD-L1. The results showed that the bispecific antibody was able to bind to human CD27 (Figure 7A) and to both human and mouse PD-L1 (Figure 7B).

The nucleotide sequence encoding the heavy chain of the 1F5 mAb x anti-PD-Ll scFv agonist construct is shown in SEQ ID NO: 64. The nucleotide sequence encoding the light chain of the 1F5 mAb x anti-PD-Ll scFv agonist construct is shown in SEQ ID NO: 65. The amino acid sequence of the heavy chain of the 1F5 mAb x anti-PD-Ll scFv agonist construct is shown in SEQ ID NO: 66. The amino acid sequence of the light chain of the 1F5 mAb x anti-PD-Ll scFv agonist construct is shown in SEQ ID NO: 65. The amino acid sequences of the VH and VL regions of the 1F5 mAb included in the construct are shown in SEQ ID NOs: 1 and 2, respectively. The VH CDR1, 2 and 3 of 1F5 are shown in SEQ ID NOs: 17, 18 and 19, respectively. The VL CDR1, 2 and 3 of 1F5 are shown in SEQ ID NOs: 20, 21 and 22, respectively. The amino acid sequences of the VH and VL regions of the anti-PD-L1 scFv used in the construct are shown in SEQ ID NOs: 32 and 33, respectively. The VH CDR1, 2 and 3 of anti-PD-Ll are shown in SEQ ID NOs: 58, 59 and 60, respectively. The VL CDR1, 2 and 3 of anti-PD-Ll are shown in SEQ ID NOs: 61, 62 and 63, respectively.

### Example 9: Immune Correlates Analysis for Anti-CD27 x Anti-PD-L1 Agonist

In this example, immune correlates were examined in mice inoculated with murine B-cell leukemia lymphoma (BCL1) cells and treated with the 1F5 mAb x anti-PDL-1 scFv bispecific agent, as compared to mice treated with anti-CD27 or anti-PD-L1 alone or in combination, to assess the activity of the bispecific agent relative to each component alone or in combination.

hCD27-Transgenic balb/c mice were inoculated intravenously with 5 × 10⁶ BCL1 cells on day 0, and were injected intraperitoneally on day 5 with 0.2 mg of either (i) anti-PD-L1 antibody; (ii) anti-CD27 antibody (1F5); (iii) these two mAbs in combination; or (iv) the 1F5 mAb x anti-PD-Ll scFv bispecific agent. Spleens were collected on day 11 for assessment. One-way analysis of variance (ANOVA) was used to compare between each treatment and saline control.

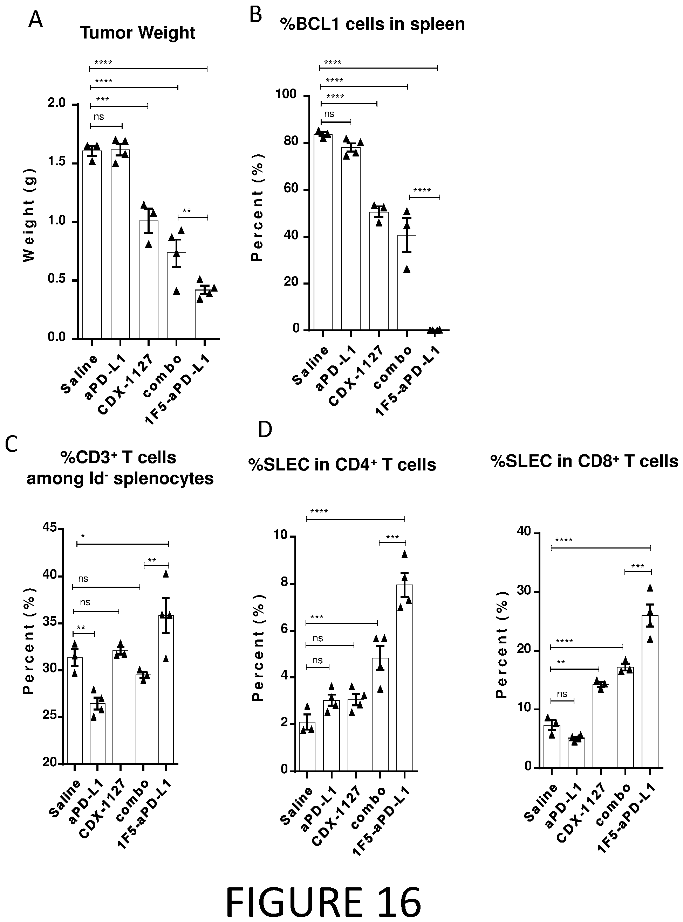

The results are shown in Figures 16A-D. Figure 16A shows spleen weights in grams, as a measure of tumor weight, and demonstrates that mice treated with the 1F5 mAb x anti-PD-L1 scFv bispecific agent had the lightest tumor load as compared to mice treated with either anti-CD27 or anti-PD-Ll alone, or with both antibodies in combination. Thus, the bispecific agent exhibited a synergistic effect that was greater than the additive effect of simply co-administering the two antibodies.

Figure16B shows BCL1 cells stained with an anti-idiotype antibody and analyzed by flow cytometry, expressed as the % of BCL1 cells in the spleen. Again, mice treated with the 1F5 mAb x anti-PD-Ll scFv bispecific agent had the lowest % of BCL1 cells in the spleen of the four treatment groups and treatment with the bispecific agent again showed a synergistic effect that was greater than the additive effect of simply co-administering the two antibodies.

Figure 16C shows the percentage of CD3⁺ T cells among non-BCLl splenocytes, whereas Figure 16D shows the percentage of CD4⁺ or CD8⁺ T cells with effector phenotype (defined by CD44⁺CD62L⁻KLRG1⁺). Mice treated with the 1F5 mAb x anti-PD-Ll scFv bispecific agent had the highest % of CD3⁺ T cells among non-BCLl splenocytes of the four treatment group, as well as the highest % of percentage of CD4⁺ or CD8⁺ T cells of the four treatment group. Moreover, treatment with the 1F5 mAb x anti-PD-Ll scFv bispecific agent again showed a synergistic effect on the percentage of CD3⁺ T cells and the percentage of CD4⁺ or CD8⁺ T cells, as compared to treatment with either anti-CD27 or anti-PD-Ll alone or with both antibodies in combination.

### Example 10: Effect of Anti-CD27 × Anti-PD-L1 Agonist in Tumor Model

In this example, the BCL1 tumor model was used to examine the effect of treatment with the 1F5 mAb x anti-PDL-1 scFv bispecific agent, as compared to treatment with anti-CD27 or anti-PD-Ll alone or in combination.

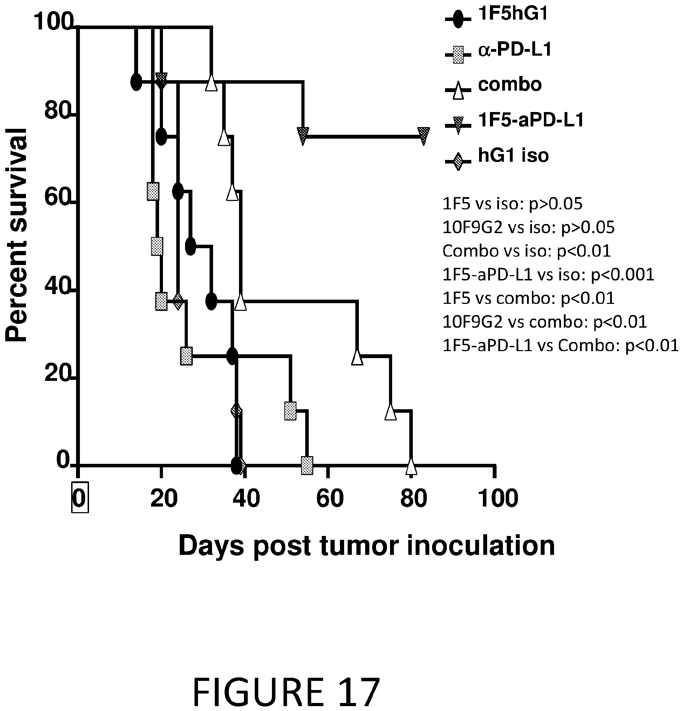

hCD27-Transgenic balb/c mice were inoculated intravenously with 5 × 10⁶ BCL1 cells on day 0, and injected intraperitoneally on day 5 with 0.2 mg of anti-PD-Ll antibody, anti-CD27 antibody (1F5), these two mAbs in combination or the bispecific 1F5 mAb x anti-PD-L1 scFv agent. Survival was monitored daily and compared between treatment groups using the Mantel-Cox test. The results are shown in Figure 17, which demonstrates that survival was significantly greater with treatment with the 1F5 mAb x anti-PD-Ll scFv bispecific agent than with treatment with either anti-PD-Ll antibody or anti-CD27 antibody (1F5) alone, or treatment with both antibodies in combination. Again, a synergistic effect was observed using the 1F5 mAb x anti-PD-Ll scFv bispecific agent that was greater that the additive effect of simply co-administering the anti-CD27 and anti-PD-Ll antibodies in combination.

**SUMMARY OF SEQUENCE LISTING**

| | |
|---|---|
| 1 | 1F5 VH amino acid sequence |
| | QVQLVESGGGVVQPGRSLRLSCAASGFTFSSYDMHWVRQAPGKGLEWVAV IWYDGSNKYYADSVKGRFTISRDNSKNTLYLQMNSLRAEDTAVYYCARGS GNWGFFDYWGQGTLVTVSS |
| 2 | 1F5 VL amino acid sequence |
| | DIQMTQSPSSLSASVGDRVTITCRASQGISRWLAWYQQKPEKAPKSLIYA ASSLQSGVPSRFSGSGSGTDFTLTISSLQPEDFATYYCQQYNTYPRTFGQ GTKVEIK |
| 3 | 2C2 VH amino acid sequence |
| | QVQLVESGGGVVQPGRSLRLSCAASGFTFSSYDIHWVRQAPGKGLEWVAV IWNDGSNKYYADSVKGRFTISRDNSTNSLFLQMNSLRAEDTAVYYCVGGT ADLEHWDQGTLVTVSS |
| 4 | 2C2 VL amino acid sequence |
| | DIQMTQSPSSLSASVGDRVTITCRASQGISSWLAWYQQKPEKAPKSLIYA ASSLQSGVPSRFSGSGSGTDFTLTISSLQPEDFATYYCQQYNSYPLTFGG GTKVEIK |
| 5 | 3H12 VH amino acid sequence |
| | QVQLVESGGGVVQPGRSLRLSCATSGFTFSSYDMHWVRQAPGKGLEWVAV IWYDGSNKYYADSVKGRFTISRDNSKNTLYLQMNSLGDEDTAVYYCARGS GNWGFFDYWGQGTLVTVSS |
| 6 | 3H12 VL amino acid sequence |
| | DIQMTQSPSSLSASVGDRVTITCRASQGISRWLAWYQQKPEKAPKSLIYA ASSLQSGVPSRFSGSGSGTDFTLTISSLQPEDFATYYCQQYNTYPRTFGQ GTKVEIK |
| 7 | 2G9 VH amino acid sequence |
| | QVQLVESGGGVVQPGRSLRLSCAASGFTLSSHDIHWVRQAPGKGLEWVAV IWNDGSNKYYADSVKGRFTISRDNSTNSLFLQMNSLRAEDTAVYYCVRGT ADLEHWDQGTLVTVSS |
| 8 | 2G9 VL amino acid sequence |
| | DIQMTQSPSSLSASVGDRVTITCRASQGISSWLAWYQQKPEKAPKSLIYA ASSLQSGVPSRFSGSGSGTDFTLTISSLQPEDFATYYCQQYNSYPLTFGG GTKVEIK |
| 9 | 1H8 VH amino acid sequence |
| | QVQLVESGGGVVQPGRSLRLSCAASGFTFNIYDMHWVRQAPGKGLEWVAV IWYDGSNQYYADSVKGRFTISRDNSKNTLYLQMNILRAEDTAVYYCARGT HWGYFDYWGQGTLVTVSS |
| 10 | 1H8 VL amino acid sequence |
| | DIQMTQSPSSLSASVGDRVTITCRASQGISSWLAWYQQKPEKAPKSLIYA ASNLQSGVPSRFSGSGSGTDFTLTISSLQPEDFATYYCQQYNSYPRTFGQ GTKVEIK |
| 11 | 3A10 VH amino acid sequence |
| | QVQLVESGGGVVQPGRSLRLSCAASGFTFSHYGMHWVRQAPGKGPEWVAI IWYDGSNKYYADSVKGRFTISRDNSKNTLDLQMNSLRAEDTAVYYCARDG WTTMVRGLNVFDIWGQGTMVTVSS |
| 12 | 3A10 VL amino acid sequence |
| | DIQMTQSPSSLSASVGDRVTITCRASQDISSWLAWYQQKPEKAPKSLIYA ASSLQSGVPSRFSGSGSGTDFTLTISSLQPEDFATYYCQQYNSYPPTFGQ GTRLEIK |
| 13 | 3H8 VH amino acid sequence |
| | EVQLVESGGGLVQPGGSLRLSCAASGFTFSSYWMAWVRQAPGKGLEWLGN IKQDGSEKYYVDSVKGRFTISRDNAKNSLYLQMNSLRAEDTAVYYCVREL GMDWYFDLWGRGTLVTVSS |
| 14 | 3H8 VL amino acid sequence |
| | EIVLTQSPATLSLSPGERATLSCRASQSVDSYLAWYQQKPGQAPRLLIYD ASNRATGIPARFSGSGSGTDFTLTISNLEPEDFAVYYCQQRSNWPPTFGQ GTKVEIK |
| 15 | 1G5 VH amino acid sequence |
| | QVQLVESGGGVVQPGRSLRLSCAASGFSFSSYGMHWVRQAPGKGLEWVAL LWYDGSHKDFADSVKGRFTISRDNSKNTLDLQMNSLRAEDTAVYYCAREG LAVPGHWYFDLWGRGTLVTVSS |
| 16 | 1G5 VL amino acid sequence |
| | AIQLTQSPSSLSASVGDRVTITCRASQGISSALAWYQQKPGKAPKLLIYD ASSLESGVPSRFSGSGSGTDFTLTISSLQPEDFATYYCQQFNTYPRTFGQ GTKVEIK |
| 17 | 1F5 VH CDR1 a.a. sequence |
| | GFTFSSYD |
| 18 | 1F5 VH CDR2 a.a. sequence |
| | IWYDGSNK |
| 19 | 1F5 VH CDR3 a.a. sequence |
| | ARGSGNWGFFDY |
| 20 | 1F5 VL CDR1 a.a. sequence |
| | QGISRW |
| 21 | 1F5 VL CDR2 a.a. sequence |
| | AAS |
| 22 | 1F5 VL CDR3 a.a. sequence |
| | QQYNTYPRT |
| 23 | 2C2 VH CDR1 a.a. sequence |
| | GFTFSSYD |
| 24 | 2C2 VH CDR2 a.a. sequence |
| | IWNDGSNK |
| 25 | 2C2 VH CDR3 a.a. sequence |
| | VGGTADLEHWDQ |
| 26 | 2C2 VL CDR1 a.a. sequence |
| | QGISSW |
| 27 | 2C2 VL CDR2 a.a. sequence |
| | AAS |
| 28 | 2C2 VL CDR3 a.a. sequence |
| | QQYNSYPLT |
| 29 | Human CD27 amino acid sequence |
| | 1 marphpwwlc vlgtlvglsa tpapkscper hywaqgklcc qmcepgtflv kdcdqhrkaa 61 qcdpcipgvs fspdhhtrph cescrhcnsg llvrnctita naecacrngw qcrdkectec 121 dplpnpslta rssqalsphp qpthlpyvse mleartaghm qtladfrqlp artlsthwpp 181 qrslcssdfi rilvifsgmf lvftlagalf lhqrrkyrsn kgespvepae pcryscpree 241 egstipiqed yrkpepacsp |
| 30 | Human CD70 amino acid sequence |
| | 1 mpeegsgcsv rrrpygcvlr aalvplvagl viclvvciqr faqaqqqlpl eslgwdvael 61 qlnhtgpqqd prlywqggpa lgrsflhgpe ldkgqlrihr dgiymvhiqv tlaicsstta 121 srhhpttlav gicspasrsi sllrlsfhqg ctiasqrltp largdtlctn ltgtllpsrn 181 tdetffgvqw vrp |
| 31 | CD70ECD |
| | AQQQLPLESLGWDVAELQLNHTGPQQDPRLYWQGGPALGRSFLHGPELDKGQLRIHRDGIYMVHIQVTL AICSSTTASRHHPTTLAVGICSPASRSISLLRLSFHQGCTIASQRLTPLARGDTLCTNLTGTLLPSRNTDETFF GVQWVRP |
| 32 | Anti-PD-L1 VH |
| | EVQLLESGGGLVQPGGSLRLSCAASGFTFSSYIMMWVRQAPGKGLEWV SSIYPSGGITFYADTVKGRFTISRDNSKNTLYLQMNSLRAEDTAVYYC ARIKLGTVTTVDYWGQGTLVTVSS |
| 33 | Anti-PD-L1 VL |
| | QSALTQPASVSGSPGQSITISCTGTSSDVGGYNYVSWYQQHPGKAPKL MIYDVSNRPSGVSNRFSGSKSGNTASLTISGLQAEDEADYYCSSYTSS STRVFGTGTKVTVL |
| 34 | Anti-TIM1 VH |
| | MEFGLSWLFLVAILKGVQCQVQLVESGGGVVQPGRSLRLSCAAS GFIFSRYGMHWVRQAPGKGLKWVAVIWYDGSNKLYADSVKGRFTIS RDNSKNTLYLQMNSLRAEDTAVYYCARDYYDNSRHHWGFDY WGQGTLVTVSSA |
| 35 | Anti-TIM1 VL |
| | METPAQLLFLLLLWLPDTTGDIVMTQTPLSLPVTPGEPASISCRSSRS LLDSDDGNTYLDWYLQKPGQSPQLLIYTLSYRASGVPDRFSGSGSGT DFTLKISRVEAEDVGVYYCMQRVEFPITFGQGTRLEIKR |
| 36 | SIINFEKL |
| 37 | 2G9 VH CDR1 a.a. sequence |
| | GFTLSSHD |
| 38 | 2G9 VH CDR3 a.a. sequence |
| | VRGTADLEHWDQ |
| 39 | 1H8 VH CDR1 a.a. sequence |
| | GFTFNIYD |
| 40 | 1H8 VH CDR2 a.a. sequence |
| | IWYDGSNQ |
| 41 | 1H8 VH CDR3 a.a. sequence |
| | ARGTHWGYFDY |
| 42 | 1H8 VL CDR3 a.a. sequence |
| | QQYNSYPRT |
| 43 | 3A10 VH CDR1 a.a. sequence |
| | GFTFSHY |
| 44 | 3A10 VH CDR3 a.a. sequence |
| | ARDGWTTMVRGLNVFDI |
| 45 | 3A10 VL CDR1 a.a. sequence |
| | QDISSW |
| 46 | 3A10 VL CDR3 a.a. sequence |
| | QQYNSYPPT |
| 47 | 3H8 VH CDR1 a.a. sequence |
| | GFTFSSY |
| 48 | 3H8 VH CDR2 a.a. sequence |
| | IKQDGSEK |
| 49 | 3H8 VH CDR3 a.a. sequence |
| | VRELGMDWYFDL |
| 50 | 3H8 VL CDR1 a.a. sequence |
| | QSVDSY |
| 51 | 3H8 VL CDR2 a.a. sequence |
| | DAS |
| 52 | 3H8 VL CDR3 a.a. sequence |
| | QQRSNWPPT |
| 53 | 1G5 VH CDR1 a.a. sequence |
| | GFSFSSY |
| 54 | 1G5 VH CDR2 a.a. sequence |
| | LLWYDGSHK |
| 55 | 1G5 VH CDR3 a.a. sequence |
| | AREGLAVPGHWYFDL |
| 56 | 1G5 VL CDR1 a.a. sequence |
| | QGISSA |
| 57 | 1G5 VL CDR3 a.a. sequence |
| | QQFNTYPRT |
| 58 | Anti-PD-L1 VH CDR1 a.a. sequence |
| | SYIMM |
| 59 | Anti-PD-L1 VH CDR2 a.a. sequence |
| | SIYPSGGITFYADTVKG |
| 60 | Anti-PD-L1 VH CDR3 a.a. sequence |
| | IKLGTVTTVDY |
| 61 | Anti-PD-L1 VL CDR1 a.a. sequence |
| | TGTSSDVGGYNYVS |
| 62 | Anti-PD-L1 VL CDR2 a.a. sequence |
| | DVSNRPS |
| 63 | Anti-PD-L1 VL CDR3 a.a. sequence |
| | SSYTSSSTRV |
| 64 | 1F5xαPD-L1 Heavy Chain n.t. sequence |
| | caggtgcagctggtggagtctgggggaggcgtggtccagcctgggaggtccctgagactctcctgtgcagcgtctgg attcaccttcagtagttatgacatgcactgggtccgccaggctccaggcaaggggctggagtgggtggcagttatatg gtatgatggaagtaataaatactatgcagactccgtgaagggccgattcaccatctccagagacaattccaagaaca cgctgtatctccaaatgaacagcctgagagccgaggacacggctgtgtattactgtgcgagaggtagtggtaactgg ggtttctttgactactggggccagggaaccctggtcaccgtctcctcagctagcaccaagggcccatcggtcttcccc tggcaccctcctccaagagcacctctgggggcacagcggccctgggctgcctggtcaaggactacttccccgagccg gtgacggtgtcgtggaactcaggcgccctgaccagcggcgtgcacaccttcccggctgtcctacagtcctcaggactc tactccctcagcagcgtggtgaccgtgccctccagcagcttgggcacccagacctacatctgcaacgtgaatcacaa gcccagcaacaccaaggtggacaagaaagttgagcccaaatcttgtgacaaaactcacacatgcccaccgtgccca gcacctgaactcctggggggaccgtcagtcttcctcttccccccaaaacccaaggacaccctcatgatctcccggacc cctgaggtcacatgcgtggtggtggacgtgagccacgaagaccctgaggtcaagttcaactggtacgtggacggcgt ggaggtgcataatgccaagacaaagccgcgggaggagcagtacaacagcacgtaccgtgtggtcagcgtcctcac cgtcctgcaccaggactggctgaatggcaaggagtacaagtgcaaggtctccaacaaagccctcccagcccccatc gagaaaaccatctccaaagccaaagggcagccccgagaaccacaggtgtacaccctgcccccatcccgggatgag ctgaccaagaaccaggtcagcctgacctgcctggtcaaaggcttctatcccagcgacatcgccgtggagtgggagag caatgggcagccggagaacaactacaagaccacgcctcccgtgctggactccgacggctccttcttcctctacagca agctcaccgtggacaagagcaggtggcagcaggggaacgtcttctcatgctccgtgatgcatgaggctctgcacaac cactacacgcagaagagcctctccctgtctccgggtaaaggctcgagcgggggaggaggtagccagtccgccctga cccagcctgcctccgtgtctggctcccctggccagtccatcaccatcagctgcaccggcacctccagcgacgtgggcg gctacaactacgtgtcctggtatcagcagcaccccggcaaggcccccaagctgatgatctacgacgtgtccaaccgg ccctccggcgtgtccaacagattctccggctccaagtccggcaacaccgcctccctgaccatcagcggactgcaggc agaggacgaggccgactactactgctcctcctacacctcctccagcaccagagtgttcggcaccggcacaaaagtga ccgtgctgggagggggcggttccggaggaggcggcagcgggggaggaggtagcgaggtgcagctgctggaatcc ggcggaggactggtgcagcctggcggctccctgagactgtcttgcgccgcctccggcttcaccttctccagctacatc atgatgtgggtgcgacaggcccctggcaagggcctggaatgggtgtcctccatctacccctccggcggcatcaccttc tacgccgacaccgtgaagggccggttcaccatctcccgggacaactccaagaacaccctgtacctgcagatgaactc cctgcgggccgaggacaccgccgtgtactactgcgcccggatcaagctgggcaccgtgaccaccgtggactactgg ggccagggcaccctggtgacagtgtcctcc |
| 65 | 1F5xαPD-L1 Light Chain n.t. sequence |
| | gacatccagatgacccagtctccatcctcactgtctgcatctgtaggagacagagtcaccatcacttgtcgggcgagt cagggtattagcaggtggttagcctggtatcagcagaaaccagagaaagcccctaagtccctgatctatgctgcatcc agtttgcaaagtggggtcccatcaaggttcagcggcagtggatctgggacagatttcactctcaccatcagcagcctg cagcctgaagattttgcaacttattactgccaacagtataatacttaccctcggacgttcggccaagggaccaaggtg |
| | gaaatcaaacgtacggtggcggcgccatctgtcttcatcttcccgccatctgatgagcagttgaaatctggaactgcct ctgttgtgtgcctgctgaataacttctatcccagagaggccaaagtacagtggaaggtggataacgccctccaatcgg gtaactcccaggagagtgtcacagagcaggacagcaaggacagcacctacagcctcagcagcaccctgacgctga gcaaagcagactacgagaaacacaaagtctacgcctgcgaagtcacccatcagggcctgagctcgcccgtcacaaa gagcttcaacaggggagagtgt |
| 66 | 1F5xαPD-L1 Heavy Chain a.a. sequence |
| | qvqlvesgggvvqpgrslrlscaasgftfssydmhwvrqapgkglewvaviwydgsnkyyadsvkgrftisr dnskntlylqmnslraedtavyycargsgnwgffdywgqgtlvtvssastkgpsvfplapsskstsggtaalgcl vkdyfpepvtvswnsgaltsgvhtfpavlqssglyslssvvtvpssslgtqtyicnvnhkpsntkvdkkvepks cdkthtcppcpapellggpsvflfppkpkdtlmisrtpevtcvvvdvshedpevkfnwyvdgvevhnaktkp reeqynstyrvvsvltvlhqdwlngkeykckvsnkalpapiektiskakgqprepqvytlppsrdeltknqvsl tclvkgfypsdiavewesngqpennykttppvldsdgsfflyskltvdksrwqqgnvfscsvmhealhnhyt qkslslspgkgssggggsqsaltqpasvsgspgqsitisctgtssdvggynyvswyqqhpgkapklmiydvsn rpsgvsnrfsgsksgntasltisglqaedeadyycssytsssstrvfgtgtkvtvlggggsggggsggggsevqlles ggglvqpggslrlscaasgftfssyimmwvrqapgkglewvssiypsggitfyadtvkgrftisrdnskntlylq mnslraedtavyycariklgtvttvdywgqgtlvtvss |
| 67 | 1F5xαPD-L1 Light Chain a.a. sequence |
| | Diqmtqspsslsasvgdrvtitcrasqgisrwlawyqqkpekapksliyaasslqsgvpsrfsgsgsgtdftltis slqpedfatyycqqyntyprtfgqgtkveikrtvaapsvfifppsdeqlksgtasvvcllnnfypreakvqwkv dnalqsgnsqesvteqdskdstyslsstltlskadyekhkvyacevthqglsspvtksfnrgec |
| 68 | Nivolumab Heavy Chain a.a. sequence |
| | QVQLVESGGG VVQPGRSLRL DCKASGITFS NSGMHWVRQA PGKGLEWVAV IWYDGSKRYY ADSVKGRFTI SRDNSKNTLF LQMNSLRAED TAVYYCATND DYWGQGTLVT VSSASTKGPS VFPLAPCSRS TSESTAALGC LVKDYFPEPV TVSWNSGALT SGVHTFPAVL QSSGLYSLSS VVTVPSSSLG TKTYTCNVDH KPSNTKVDKR VESKYGPPCP PCPAPEFLGG PSVFLFPPKP KDTLMISRTP EVTCVVVDVS QEDPEVQFNW YVDGVEVHNA KTKPREEQFN STYRVVSVLT VLHQDWLNGK EYKCKVSNKG LPSSIEKTIS KAKGQPREPQ VYTLPPSQEE MTKNQVSLTC LVKGFYPSDI AVEWESNGQP ENNYKTTPPV LDSDGSFFLY SRLTVDKSRW QEGNVFSCSV MHEALHNHYT QKSLSLSLGK |
| 69 | Nivolumab Light Chain a.a. sequence |
| | EIVLTQSPAT LSLSPGERAT LSCRASQSVS SYLAWYQQKP GQAPRLLIYD ASNRATGIPA RFSGSGSGTD FTLTISSLEP EDFAVYYCQQ SSNWPRTFGQ GTKVEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC |
| 70 | Pembrolizumab Heavy Chain a.a. sequence |
| | QVQLVQSGVEVKKPGASVKVSCKASGYTFTNYYMYWVRQAPGQGLEWMG |
| | GINPSNGGTNFNEKFKNRVTLTTDSSTTTAYMELKSLQFDDTAVYYCARRDY RFDMGFDYWGQGTTVTVSSASTKGPSVFPLAPCSRSTSESTAALGCLVKDYFP EPVTVSWNSGALTSGVHTFPAVLQSSGLYSLSSVVTVPSSSLGTKTYTCNVDH KPSNTKVDKRVESKYGPPCPPCPAPEFLGGPSVFLFPPKPKDTLMISRTPEVTC VVVDVSQEDPEVQFNWYVDGVEVHNAKTKPREEQFNSTYRVVSVLTVLHQD WLNGKEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDKSRW QEGNVFSCSVMHEALHNHYTQKSLSLSLGK |
| **71** | Pembrolizumab Light Chain a.a. sequence |
| | EIVLTQSPATLSLSPGERATLSCRASKGVSTSGYSYLHWYQQKPGQAPRLLIYL ASYLESGVPARFSGSGSGTDFTLTISSLEPEDFAVYYCQHSRDLPLTFGGGTKV EIKRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFN RGEC |
| **72** | Durvelumab Heavy Chain a.a. sequence |
| | EVQLVESGGG LVQPGGSLRL SCAASGFTFS RYWMSWVRQA PGKGLEWVAN IKQDGSEKYY VDSVKGRFTI SRDNAKNSLY LQMNSLRAED TAVYYCAREG GWFGELAFDY WGQGTLVTVS SASTKGPSVF PLAPSSKSTS GGTAALGCLV KDYFPEPVTV SWNSGALTSG VHTFPAVLQS SGLYSLSSVV TVPSSSLGTQ TYICNVNHKP SNTKVDKRVE PKSCDKTHTC PPCPAPEFEG GPSVFLFPPK PKDTLMISRT PEVTCVVVDV SHEDPEVKFN WYVDGVEVHN AKTKPREEQY NSTYRVVSVL TVLHQDWLNG KEYKCKVSNK ALPASIEKTI SKAKGQPREP QVYTLPPSRE EMTKNQVSLT CLVKGFYPSD IAVEWESNGQ PENNYKTTPP VLDSDGSFFL YSKLTVDKSR WQQGNVFSCS VMHEALHNHY TQKSLSLSPG K |
| **73** | Durvelumab Light Chain a.a. sequence |
| | EIVLTQSPGT LSLSPGERAT LSCRASQRVS SSYLAWYQQK PGQAPRLLIY DASSRATGIP DRFSGSGSGT DFTLTISRLE PEDFAVYYCQ QYGSLPWTFG QGTKVEIKRT VAAPSVFIFP PSDEQLKSGT ASVVCLLNNF YPREAKVQWK VDNALQSGNS QESVTEQDSK DSTYSLSSTL TLSKADYEKH KVYACEVTHQ GLSSPVTKSF NRGEC |
| **74** | Ipilimumab Heavy Chain a.a. sequence |
| | QVQLVESGGGVVQPGRSLRLSCAASGFTFSSYTMHWVRQAPGKGLEWVTFIS YDGNNKYYADSVKGRFTISRDNSKNTLYLQMNSLRAEDTAIYYCARTGWLG PFDYWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSN TKVDKRVEPKSCDKTHTCPPCPAPELLGGPSVFLFPPKPKDTLMISRTPEVTCV VVDVSHEDPEVKFNWYVDGVEVHNAKTKPREEQYNSTYRVVSVLTVLHQD WLNGKEYKCKVSNKALPAPIEKTISKAKGQPREPQVYTLPPSRDELTKNQVSL TCLVKGFYPSDIAVEWESNGQPENNYKTTPPVLDSDGSFFLYSKLTVDKSRW QQGNVFSCSVMHEALHNHYTQKSLSLSPGK |
| **75** | Ipilimumab Light Chain a.a. sequence |
| | EIVLTQSPGTLSLSPGERATLSCRASQSVGSSYLAWYQQKPGQAPRLLIYGAFS RATGIPDRFSGSGSGTDFTLTISRLEPEDFAVYYCQQYGSSPWTFGQGTKVEIK RTVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNRG EC |

## Claims

1. A CD27 agonist comprising a first component that is an anti-CD27 antibody, or antigen-binding fragment thereof, linked to a second component that is an anti-PD-L1 antibody, or antigen-binding fragment thereof.

2. The CD27 agonist of claim 1, wherein
a. the anti-PD-Ll antibody, or antigen-binding fragment thereof does not bind to an Fc receptor; and
b. the CD27 agonist stimulates T cell activity without the need for Fc receptor interaction.

3. The CD27 agonist of claim 1 or 2, wherein the second component is an anti-PD-L1 scFv.

4. The CD27 agonist of claim 2 or 3, wherein the first component is an anti-CD27 antibody and the second component is an anti-PD-L1 scFv linked to the C-terminus of the heavy chain of the anti-CD27 antibody.

5. The CD27 agonist of claim 3 or 4, wherein (i) the anti-CD27 antibody comprises a VH CDR3 shown in SEQ ID NO: 19 and a VL CDR3 shown in SEQ ID NO: 22 and (ii) the anti-PD-Ll scFv comprises a VH CDR3 shown in SEQ ID NO: 60 and a VL CDR3 shown in SEQ ID NO: 63.

6. The CD27 agonist of any one of claims 3 to 5, wherein (i) the anti-CD27 antibody comprises VH CDR1, CDR2 and CDR3 shown in SEQ ID NOs: 17, 18 and 19, respectively, and VL CDR1, CDR2 and CDR3 shown in SEQ ID NOs: 20, 21 and 22, respectively, and (ii) the anti-PD-L1 scFv comprises VH CDR1, CDR2 and CDR3 shown in SEQ ID NOs: 58, 59 and 60, respectively, and VL CDR1, CDR2 and CDR3 shown in SEQ ID NOs: 61, 62 and 63, respectively.

7. The CD27 agonist of any one of claims 3 to 6, wherein (i) the anti-CD27 antibody comprises VH and VL chains comprising the amino acid sequences shown in SEQ ID NOs: 1 and 2, respectively; and (ii) the anti-PD-L1 scFv comprises VH and VL chains comprising the amino acid sequences shown in SEQ ID NOs: 32 and 33, respectively.

8. The CD27 agonist of any one of the preceding claims, which comprises a heavy chain comprising the amino acid sequence shown in SEQ ID NO: 66 and a light chain comprising the amino acid sequence shown in SEQ ID NO: 67.

9. The CD27 agonist of claim 8, wherein the heavy chain is encoded by the nucleotide sequence shown in SEQ ID NO: 64 and the light chain is encoded by the nucleotide sequence shown in SEQ ID NO: 65.

10. The CD27 agonist of any one of the preceding claims, wherein the anti-CD27 antibody is an IgG1 antibody.

11. A pharmaceutical composition comprising the CD27 agonist of any one of claims 1-10 and a pharmaceutically acceptable carrier.

12. An *in vitro* method of stimulating T cell activity comprising contacting T cells with the CD27 agonist of any one of claims 1-10, optionally wherein stimulating T cell activity comprises stimulating IFN-gamma production.

13. The CD27 agonist of any one of claims 1 to 10 for use in a method for inducing or enhancing an immune response in a subject.

14. The CD27 agonist for use according to claim 13, wherein the subject suffers from a condition or disease in which stimulation of an immune response is desired, optionally wherein the condition or disease is cancer.

15. The CD27 agonist for use according to any one of claims 13 or 14, wherein the method further comprises administering an antigen to the subject, optionally wherein the antigen is a tumor antigen.

## Patentansprüche

1. CD27-Agonist, der eine erste Komponente umfasst, bei der es sich um einen Anti-CD27-Antikörper oder ein Antigen-bindendes Fragment davon handelt, verbunden mit einer zweiten Komponente, bei der es sich um einen Anti-PD-Ll-Antikörper oder ein Antigen-bindendes Fragment davon handelt.

2. CD27-Agonist nach Anspruch 1, wobei
a. der Anti-PD-L1-Antikörper oder das Antigen-bindende Fragment davon nicht an einen Fc-Rezeptor bindet; und
b. der CD27-Agonist die T-Zell-Aktivität stimuliert, ohne dass eine Fe-Rezeptor-Interaktion erforderlich ist.

3. CD27-Agonist nach Anspruch 1 oder 2, wobei die zweite Komponente ein Anti-PD-L1 scFv ist.

4. CD27-Agonist nach Anspruch 2 oder 3, wobei die erste Komponente ein Anti-CD27-Antikörper ist und die zweite Komponente ein Anti-PD-L1 scFv ist, das an den C-Terminus der schweren Kette des Anti-CD27-Antikörpers gebunden ist.

5. CD27-Agonist nach Anspruch 3 oder 4, wobei (i) der Anti-CD27-Antikörper ein VH CDR3, das in SEQ ID NO: 19 gezeigt ist, und ein VL CDR3, das in SEQ ID NO: 22 gezeigt ist, umfasst und (ii) der Anti-PD-L1 scFv ein VH CDR3, das in SEQ ID NO: 60 gezeigt ist, und ein VL CDR3, das in SEQ ID NO: 63 gezeigt ist, umfasst.

6. CD27-Agonist nach einem der Ansprüche 3 bis 5, wobei (i) der Anti-CD27-Antikörper VH CDR1, CDR2 und CDR3, die in SEQ ID NO: 17, 18 bzw. 19 gezeigt sind, und VL CDR1, CDR2 und CDR3, die in SEQ ID NO: 20, 21 bzw. 22 gezeigt sind, umfasst, und (ii) der Anti-PD-L1 scFv VH CDR1, CDR2 und CDR3, die in SEQ ID NO: 58, 59 bzw. 60 gezeigt sind, und VL CDR1, CDR2 und CDR3, die in SEQ ID NO: 61, 62 bzw. 63 gezeigt sind, umfasst.

7. CD27-Agonist nach einem der Ansprüche 3 bis 6, wobei (i) der Anti-CD27-Antikörper VH- und VL-Ketten umfasst, die die Aminosäuresequenzen umfassen, die in SEQ ID NOs: 1 bzw. 2 gezeigt sind; und (ii) der Anti-PD-L1 scFv VH- und VL-Ketten umfasst, die die Aminosäuresequenzen umfassen, die in SEQ ID NOs: 32 bzw. 33 gezeigt sind.

8. CD27-Agonist nach einem der vorhergehenden Ansprüche, der eine schwere Kette, die die Aminosäuresequenz umfasst, die in SEQ ID NO: 66 gezeigt ist, und eine leichte Kette, die die Aminosäuresequenz umfasst, die in SEQ ID NO: 67 gezeigt ist, umfasst.

9. CD27-Agonist nach Anspruch 8, wobei die schwere Kette durch die Nukleotidsequenz codiert ist, die in SEQ ID NO: 64 gezeigt ist, und die leichte Kette durch die Nukleotidsequenz codiert ist, die in SEQ ID NO: 65 gezeigt ist.

10. CD27-Agonist nach einem der vorhergehenden Ansprüche, wobei der Anti-CD27-Antikörper ein IgG1-Antikörper ist.

11. Pharmazeutische Zusammensetzung, die den CD27-Agonisten nach einem der Ansprüche 1 bis 10 und einen pharmazeutisch akzeptablen Träger umfasst.

12. In-vitro-Verfahren zur Stimulierung der T-Zell-Aktivität, das das Inkontaktbringen von T-Zellen mit dem CD27-Agonisten nach einem der Ansprüche 1 bis 10 umfasst, wobei die Stimulierung der T-Zell-Aktivität optional die Stimulierung der IFN-gamma-Produktion umfasst.

13. CD27-Agonist nach einem der Ansprüche 1 bis 10 zur Verwendung in einem Verfahren zur Induktion oder Verstärkung einer Immunantwort in einem Subjekt.

14. CD27-Agonist zur Verwendung nach Anspruch 13, wobei das Subjekt an einem Zustand oder einer Krankheit leidet, bei der eine Stimulation einer Immunantwort erwünscht ist, wobei der Zustand oder die Krankheit optional Krebs ist.

15. CD27-Agonist zur Verwendung nach einem der Ansprüche 13 oder 14, wobei das Verfahren ferner die Verabreichung eines Antigens an das Subjekt umfasst, wobei das Antigen optional ein Tumorantigen ist.

## Revendications

1. Agoniste de CD27 comprenant un premier composant qui est un anticorps anti-CD27, ou un fragment de liaison à l'antigène de celui-ci, lié à un deuxième composant qui est un anticorps anti-PD-L1, ou un fragment de liaison à l'antigène de celui-ci.

2. Agoniste de CD27 selon la revendication 1, dans lequel
a. l'anticorps anti-PD-L1, ou un fragment de liaison à l'antigène celui-ci, ne se lie pas à un récepteur Fc ; et
b. l'agoniste de CD27 stimule l'activité des cellules T sans nécessiter d'interaction avec le récepteur Fc.

3. Agoniste de CD27 selon la revendication 1 ou 2, dans lequel le deuxième composant est un scFv anti-PD-L1.

4. Agoniste de CD27 selon la revendication 2 ou 3, dans lequel le premier composant est un anticorps anti-CD27 et le deuxième composant est un scFv anti-PD-L1 lié à l'extrémité C-terminale de la chaîne lourde de l'anticorps anti-CD27.

5. Agoniste de CD27 selon la revendication 3 ou 4, dans lequel (i) l'anticorps anti-CD27 comprend une VH CDR3 représentée dans SEQ ID NO : 19 et une VL CDR3 représentée dans SEQ ID NO : 22 et (ii) l'anticorps anti-PD-L1 scFv comprend une VH CDR3 représentée dans la SEQ ID NO : 60 et une VL CDR3 représentée dans la SEQ ID NO : 63.

6. Agoniste de CD27 selon l'une quelconque des revendications 3 à 5, dans lequel (i) l'anticorps anti-CD27 comprend les VH CDR1, CDR2 et CDR3 représentées dans les SEQ ID NO : 17, 18 et 19, respectivement, et les VL CDR1, CDR2 et CDR3 représentées dans les SEQ ID NO : 20, 21 et 22, respectivement, et (ii) le scFv anti-PD-L1 comprend les VH CDR1, CDR2 et CDR3 représentées dans les SEQ ID NO : 58, 59 et 60, respectivement, et les VL CDR1, CDR2 et CDR3 représentées dans les SEQ ID NO : 61, 62 et 63, respectivement.

7. Agoniste de CD27 selon l'une quelconque des revendications 3 à 6, dans lequel (i) l'anticorps anti-CD27 comprend des chaînes VH et VL comprenant les séquences d'acides aminés représentées dans les SEQ ID NO : 1 et 2, respectivement ; et (ii) le scFv anti-PD-L1 comprend des chaînes VH et VL comprenant les séquences d'acides aminés représentées dans les SEQ ID NO : 32 et 33, respectivement.

8. Agoniste de CD27 selon l'une quelconque des revendications précédentes, qui comprend une chaîne lourde comprenant la séquence d'acides aminés représentée dans la SEQ ID NO : 66 et une chaîne légère comprenant la séquence d'acides aminés représentée dans la SEQ ID NO : 67.

9. Agoniste de CD27 selon la revendication 8, dans lequel la chaîne lourde est codée par la séquence nucléotidique représentée dans la SEQ ID NO : 64 et la chaîne légère est codée par la séquence nucléotidique représentée dans la SEQ ID NO : 65.

10. Agoniste de CD27 selon l'une quelconque des revendications précédentes, dans lequel l'anticorps anti-CD27 est un anticorps IgG1.

11. Composition pharmaceutique comprenant l'agoniste de CD27 selon l'une quelconque des revendications 1 à 10 et un support pharmaceutiquement acceptable.

12. Procédé *in vitro* de stimulation de l'activité des cellules T comprenant la mise en contact des cellules T avec l'agoniste de CD27 selon l'une quelconque des revendications 1 à 10, éventuellement dans lequel la stimulation de l'activité des cellules T comprend la stimulation de la production d'IFN-gamma.

13. Agoniste de CD27 selon l'une quelconque des revendications 1 à 10 pour une utilisation dans un procédé pour induire ou renforcer une réponse immunitaire chez un sujet.

14. Agoniste de CD27 pour une utilisation selon la revendication 13, dans lequel le sujet souffre d'un état ou d'une maladie dans lequel la stimulation d'une réponse immunitaire est souhaitée, éventuellement dans lequel l'état ou la maladie est le cancer.

15. Agoniste de CD27 pour une utilisation selon l'une quelconque des revendications 13 ou 14, dans lequel le procédé comprend en outre l'administration d'un antigène au sujet, éventuellement dans lequel l'antigène est un antigène tumoral.
